# EUROPEAN PATENT APPLICATION

(11) **EP 3 985 104 A1**
(43) Date of publication of application: **20.04.2022**
(21) Application number: 20822392.5
(22) Date of filing: 10.06.2020
(51) Int. Cl.: C12N 5/077, C12N 1/00, C12P 21/02

(54) **METHOD FOR PRODUCING RENAL INTERSTITIAL CELL**

(30) Priority: 11.06.2019 JP 2019108342
(71) Applicant: Kyoto University, Kyoto-shi, Kyoto 606-8501 (JP); Takeda Pharmaceutical Company Limited, Osaka-shi, Osaka 541-0045 (JP)
(72) Inventor: IKEYA, Makoto, Kyoto-shi, Kyoto 606-8501 (JP); KAMIYA, Daisuke, Kyoto-shi, Kyoto 606-8501 (JP)
(74) Representative: Jones, Nicholas Andrew
(86) International application number: PCT/JP2020/022769
(87) International publication number: WO 2020/250913

(57) **Abstract**

A method for producing renal stromal cells, comprising a step (3) of culturing renal stromal precursors in a medium comprising a platelet derived growth factor receptor agonist to obtain renal stromal cells is provided as a technique for supplying renal stromal cells. This production method can further comprise a step (2) of inducing renal stromal precursors from neural crest cells, and a step (1) of culturing pluripotent stem cells in a medium comprising a GSK3β inhibitor, a TGFβ inhibitor, and retinoic acid and/or a derivative thereof to induce neural crest cells.

## Description

### Technical Field

The present invention relates to a method and a medium for producing renal stromal cells, and a method for producing a kidney organoid using renal stromal cells.

### [Background of Invention]

Renal fibrosis, which is caused by chronic kidney disease and acute renal disease, etc., involves significant reduction in renal function and brings about the need of introduction of dialysis or renal transplantation to patients. In recent years, it has been revealed that renal stromal cells (RSCs) such as erythropoietin (EPO)-producing cells and fibroblasts are involved in renal fibrosis. Testing systems using the renal stromal cells are considered useful for elucidating the mechanism of renal fibrosis and establishing prevention and treatment methods.

Non Patent Literature 1 states that EPO-producing cells were induced from induced pluripotent stem cells (iPSCs). This induction method comprises, for example, the step of culturing iPSCs in a medium comprising activin A, CHIR99021 (a GSK3β inhibitor) and Y-27632 (a ROCK inhibitor). The EPO-producing cells obtained by the method described in Non Patent Literature 1 are of hepatic lineage and are different from renal stromal cells.

Non Patent Literature 2 states that renal progenitors were induced from iPSCs. This induction method comprises, for example, the step of culturing iPSCs in a medium comprising activin A and CHIR99021 (GSK3β inhibitor).

Any technique for inducing renal stromal cells from pluripotent stem cells such as iPSCs *in vitro* has not yet been reported.

### Citation List

### Non Patent Literature

Non Patent Literature 1: "Human pluripotent stem cell-derived erythropoietin-producing cells ameliorate renal anemia in mice", Science Translational Medicine, 2017, Vol. 9, Issue 409, eaaj2300
Non Patent Literature 2: "Cell Therapy Using Human Induced Pluripotent Stem Cell-Derived Renal Progenitors Ameliorates Acute Kidney Injury in Mice", STEM CELLS TRANSLATIONAL MEDICINE 2015;4:980-992
Non Patent Literature 3: "Deriving human ENS lineages for cell therapy and drug discovery in Hirschsprung disease", Nature, 2016, 531, 105-109
Non Patent Literature 4: "Derivation of mesenchymal stromal cells from pluripotent stem cells through a neural crest lineage using small molecule compounds with defined media", PLoS One, 2014, 9, 12
Non Patent Literature 5: "A novel efficient feeder-free culture system for the derivation of human induced pluripotent stem cells", Scientific Reports, 2014, 4, 3594
Non Patent Literature 6: "Generation of kidney organoids from human pluripotent stem cells", Nature Protocols volume 2016, 11, 1681-1692
Non Patent Literature 7: "Making a Kidney Organoid Using the Directed Differentiation of Human Pluripotent Stem Cells", Methods in Molecular Biology, 2017, 1597, 195-206

### Summary of Invention

### Technical Problem

There is a demand for a technique for supplying renal stromal cells, for example, for the construction of testing systems using renal stromal cells.

### Solution to Problem

In order to solve the problem, the present invention provides the following [1] to [31].
[1] A method for producing renal stromal cells, comprising a step (3) of culturing renal stromal precursors in a medium comprising a platelet derived growth factor receptor agonist to obtain renal stromal cells.
[2] The production method according to [1], wherein the medium further comprises a basic fibroblast growth factor and/or fibroblast growth factor 9.
[3] The production method according to [1] or [2], further comprising a step (2) of inducing renal stromal precursors from neural crest cells.
[4] The production method according to any of [1] to [3], further comprising a step (1) of culturing pluripotent stem cells in a medium comprising a GSK3β inhibitor, a TGFβ inhibitor, and retinoic acid and/or a derivative thereof to induce neural crest cells.
[4a] The production method according to [4], wherein the pluripotent stem cells are induced pluripotent stem cells.
[4b] The production method according to [4] or [4a], wherein the GSK3β inhibitor is CHIR98014 (N6-[2-[[4-(2,4-dichlorophenyl)-5-(1H-imidazol-1-yl)-2-pyrimidinyl]amino]ethyl]-3-nitro-2,6-pyridinediamine).
[4c] The production method according to any of [4] to [4b], wherein the TGFβ inhibitor is SB431542 (4-[4-(1,3-benzodioxol-5-yl)-5-(2-pyridinyl)-1H-imidazol-2-yl]-benzamide).
[5] The production method according to any of [3] to [4c], wherein the neural crest cells are hindbrain neural crest cells.
[6] The production method according to any of [1] to [5], wherein the renal stromal cells produce erythropoietin under hypoxic conditions.
[7] The production method according to any of [1] to [6], wherein the platelet derived growth factor receptor agonist is a platelet derived growth factor (PDGF).
[8] A medium for use in producing renal stromal cells, comprising a platelet derived growth factor receptor agonist.
[9] The medium according to [8], further comprising a basic fibroblast growth factor and/or fibroblast growth factor 9.
[10] The medium according to [8] or [9], wherein the platelet derived growth factor receptor agonist is a platelet derived growth factor (PDGF).
[11] A method for producing a kidney organoid, comprising the step of coculturing renal stromal cells or renal stromal precursors, and an intermediate mesoderm.
[12] The production method according to [11], wherein
   the renal stromal cells or the renal stromal precursors are obtained by a method for producing renal stromal cells, comprising a step (3) of culturing renal stromal precursors in a medium comprising a platelet derived growth factor receptor agonist to obtain renal stromal cells, or
   obtained by a step (2) of inducing renal stromal precursors from neural crest cells.
[13] A method for screening for a substance for the prevention or treatment of renal fibrosis, comprising the steps of:
   culturing renal stromal cells obtained by a production method according to any of [1] to [7] in the presence of a substance that induces the fibrosis of the cells to provide a cell population comprising fibrotic renal stromal cells;
   culturing the cell population in the presence of and in the absence of a test substance;
   measuring a degree of fibrosis of the cells in each of the cell populations thus obtained; and
   selecting a test substance that has reduced the degree of fibrosis of the cells in the cell population cultured in the presence of the test substance as compared with the cell population cultured in the absence of the test substance.
[13a] The method according to [13], wherein the substance that induces the fibrosis is TGFβ (transforming growth factor β).
[14] A method for determining a biomarker for renal fibrosis, comprising the steps of:
   culturing renal stromal cells obtained by a production method according to any of [1] to [7] in the presence of a substance that induces the fibrosis of the cells to provide a cell population comprising fibrotic renal stromal cells;
   identifying a substance contained in the culture solution of the cell population; and
   comparing the identified substance with a substance contained in a body fluid of a mammal having renal fibrosis to determine an identical substance.
[14a] The method according to [14], wherein the substance that induces the fibrosis is TGFβ.
[15] A method for producing a medicament for the prevention or treatment of kidney damage containing renal stromal precursors, the production method comprising a step (2) of inducing renal stromal precursors from neural crest cells.
[16] A method for producing a medicament for the prevention or treatment of kidney damage containing renal stromal cells, the production method comprising a step (3) of culturing renal stromal precursors in a medium comprising a platelet derived growth factor receptor agonist to obtain renal stromal cells.
[17] The production method according to [15] or [16], further comprising a step (1) of culturing pluripotent stem cells in a medium comprising a GSK3β inhibitor, a TGFβ inhibitor, and retinoic acid and/or a derivative thereof to induce neural crest cells.
[18] A medicament comprising renal stromal precursors derived from pluripotent stem cells and/or renal stromal cells derived from pluripotent stem cells.
[18a] The medicament according to [18] for the prevention or treatment of kidney damage.
[19] A prophylactic or therapeutic agent for kidney damage, comprising renal stromal precursors derived from pluripotent stem cells and/or renal stromal cells derived from pluripotent stem cells.
[20] A method for preventing or treating kidney damage, comprising the step of administering renal stromal precursors derived from pluripotent stem cells and/or renal stromal cells derived from pluripotent stem cells to a subject.
[21] Renal stromal precursors derived from pluripotent stem cells and/or renal stromal cells derived from pluripotent stem cells for use in the prevention or treatment of kidney damage.
[22] Use of renal stromal precursors derived from pluripotent stem cells and/or renal stromal cells derived from pluripotent stem cells for the production of a medicament for the prevention or treatment of kidney damage.
[23] Renal stromal cells obtainable by culturing renal stromal precursors in a medium comprising a platelet derived growth factor receptor agonist.
[23a] The renal stromal cells according to [23], wherein the platelet derived growth factor receptor agonist is a platelet derived growth factor (PDGF).
[23b] The renal stromal cells according to [23] or [23a], wherein the medium further comprises a basic fibroblast growth factor and/or fibroblast growth factor 9.
[23c] The renal stromal cells according to any of [23] to [23b], wherein the renal stromal cells express CD73 and PDGFRβ and produce erythropoietin under hypoxic conditions.
[23d] The renal stromal cells according to [23c], wherein the renal stromal cells further express desmin and vimentin.
[24] Renal stromal precursors obtainable by culturing neural crest cells in a medium comprising StemPro(R) MSC SFM Xenofree.
[24a] The renal stromal precursors according to [24], wherein the renal stromal precursors express FOXD1, CD73 and PDGFRβ and do not produce erythropoietin.
[25] A kidney organoid comprising renal stromal cells and/or renal stromal precursors according to any of [23] to [24a].
[26] An inducer of differentiation into renal stromal cells, comprising a platelet derived growth factor receptor agonist.
[26a] The inducer of differentiation into renal stromal cells according to [26], wherein the platelet derived growth factor receptor agonist is a platelet derived growth factor (PDGF).
[27] Renal stromal precursors derived from induced pluripotent stem cells.
[28] A method for producing renal stromal precursors, comprising a step (2) of inducing renal stromal precursors from neural crest cells.
[29] Renal stromal cells derived from induced pluripotent stem cells.
[30] An inducer of differentiation into renal stromal cells, comprising STEMdiff APEL2 medium (STEMCELL Technologies Inc., ST-05275).
[31] A method for producing renal stromal cells, comprising the step of culturing renal stromal precursors in STEMdiff APEL2 medium (STEMCELL Technologies Inc., ST-05275) .

### [Definition]

In the present invention, the term "platelet derived growth factor receptor agonist" means a substance that may bind to a platelet derived growth factor receptor (PDGFR) and mediate signal transduction starting with the phosphorylation of the receptor.

"Platelet derived growth factor receptor (PDGFR)" includes two types of subtypes, PDGFRα and PDGFRβ. PDGFR forms a dimer through binding to a ligand such as a platelet derived growth factor (PDGF), and the dimer includes three types of combinations, PDGFR-αα, PDGFR-αβ and PDGFR-ββ**.** Upon binding of the ligand to PDGFR, a tyrosine residue of the PDGFR undergoes
autophosphorylation and in turn serves as a binding site for a signal transduction molecule having an SH2 domain (PLC-γ, Grb2, PI3K, etc.) so that signals are transduced downstream.

The platelet derived growth factor receptor agonist can be an antibody, a peptide, a low-molecular compound, or the like and is preferably PDGF.

"Renal stromal cells (RSCs)" are CD73-positive and PDFGRβ-positive cells and can produce erythropoietin (hereinafter, referred to as "EPO" in the present specification; "EPO" means erythropoietin protein unless otherwise specified).

The term "Fibrosis" of renal stromal cells means that renal stromal cells that are not αSMA-positive become αSMA-positive cells. The phrase "renal stromal cells become αSMA-positive" means that the cells differentiate into myofibroblasts. This differentiation can be induced by the TGFβ1 stimulation of renal stromal cells. The differentiation may involve cellular hypertrophy. The myofibroblasts are αSMA-positive.

"Renal stromal precursors (RSPs)" are FOXD1-positive, CD73-positive and PDGFRβ-positive cells and do not produce EPO.

"Neural crest cells (NCCs)" are cells that develop from between the neuroectoderm and the epidermal ectoderm when the neural tube is formed from the neural plate during early development. These cells have multipotency to differentiate into many types of cells such as nerve cells, glial cells, mesenchymal stromal cells, bone cells, chondrocytes, corneal cells and pigment cells, and the ability to self-proliferate. The neural crest cells are SOX10-positive.

"Hindbrain neural crest cells (hindbrain NCCs: hNCCs)" are posteriorly shifted NCCs and are SOX10-positive, NGFR-positive, HOXB4-positive, and erythropoietin (EPO)-positive in terms of neural crest cell markers.

"Posterior shift" is the movement of the anterior-posterior axis nearer to the posterior side during development. In the present invention, the posterior shift refers to the induction of midbrain neural crest cells (midbrain NCCs) obtained by the differentiation of pluripotent stem cells into hindbrain neural crest cells (hindbrain NCCs).

"Intermediate mesoderm" is an embryo that develops from the mesoderm in the development of an individual, is cells that may differentiate into pronephros, mesonephros, mesonephric duct, metanephros, adrenal cortex and genital gland, and is OSR1 (odd-skipped related 1)-positive.

"Kidney organoid" is a three-dimensional structure that constitutes a kidney tissue *in vivo* and contains at least one or more cell populations.

"TGFβ inhibitor" is a substance having inhibitory activity against TGFβ (transforming growth factor β). TGFβ is a cytokine binding to two types of serine/threonine protein kinase receptors and controls cell proliferation, cell differentiation and cell death, etc. via signal transduction, mainly, for activating Smad (R-Smad). Examples of the substance having TGFβ inhibitory activity include substances inhibiting the binding of TGFβ to its receptor, and substances inhibiting downstream signals after the binding of TGFβ to its receptor. Examples of the downstream signals include the phosphorylation of TGFβI receptor by TGFβII receptor, and the phosphorylation of Smad by phosphorylated TGFβI receptor. The term "TGFβ inhibitor" used in the present invention is not particularly limited as long as the TGFβ inhibitor has TGFβ inhibitory activity.

"GSK3β inhibitor" is a substance having inhibitory activity against GSK3β (glycogen synthase kinase 3β). GSK3 (glycogen synthase kinase 3) is a serine/threonine protein kinase and involved in many signaling pathways associated with the production of glycogen, apoptosis, maintenance of stem cells, etc. GSK3 has the 2 isoforms α and β. The term "GSK3β inhibitor" used in the present invention is not particularly limited as long as the GSK3β inhibitor has GSK3β inhibitory activity. The GSK3β inhibitor may be a substance having both GSK3β inhibitory activity and GSK3α inhibitory activity.

The term "culture" refers to maintenance, proliferation (growth), and/or differentiation of cells in *in vitro* environment. "Culturing" means maintaining cells and/or allowing the cells to proliferate (grow) and/or differentiate outside the tissue or the body, for example, in a cell culture dish or a flask.

The term "cell population" means two or more cells of the same type or different types. "Cell population" also means a mass of cells of the same type or different types.

The term "adherent culture" means culture in a state where cells are attached to a container, for example, in a state where cells are attached to a cell culture dish or a flask made of a sterilized plastic (or coated plastic) in the presence of an appropriate medium.

The term "suspension culture" means culture in a state where cells are dispersed in an appropriate medium without being attached to a container.

The term "maintenance culture" or "sustain" means the culture of a desired cell population with its cell number maintained. The maintenance of the cell number may be achieved by the survival of cells without proliferation or may be achieved by the balance between an increased number of cells by proliferation and a decreased number of cells by death. The maintenance of the cell number does not require cells to be maintained at completely the same number. Substantially the same number of cells can be maintained in light of the object of the present invention.

The term "expansion culture" or "expand" means culture with the aim of allowing a desired cell population to proliferate to increase the number of cells. The increase in cell number can be achieved through an increased number of cells by proliferation exceeding a decreased number of cells by death, and does not require the proliferation of all cells in the cell population. The increase in the number of cells may be 1.1 times, 1.2 times, 1.5 times, 2 times, 3 times, 4 times, 5 times, 6 times, 7 times, 8 times, 9 times, 10 times, 15 times, 20 times, or 30 times or more as compared with the number of cells before the start of expansion culture.

The term "pluripotency" means the ability to differentiate into tissues and cells having various different shapes and functions and to differentiate into cells of any lineage of the 3 germ layers. "Pluripotency" is different from "totipotency", which is the ability to differentiate into any tissue of the living body, including the placenta, in that pluripotent cells cannot differentiate into the placenta and thus do not have the ability to form an individual.

The term "multipotency" means the ability to differentiate into plural and limited numbers of linages of cells. For example, mesenchymal stem cells, hematopoietic stem cells, neural stem cells are multipotent, but not pluripotent. ENPs have multipotency to differentiate into nerve cells and glial cells.

The term "marker" is "marker protein" or "marker gene" and means a protein that is specifically expressed on cell surface, in cytosol, and/or in nucleus of a predetermined cell type, or a gene thereof. The marker may be a positive selection marker or a negative selection marker. Preferably, the marker is a cell surface marker. Particularly, a cell surface-positive selection marker allows concentration, isolation, and/or detection of living cells.

The marker protein can be detected by use of immunological assay, for example, ELISA, immunostaining, or flow cytometry, using an antibody specific for the marker protein. An antibody that binds to a specific amino acid sequence of the marker protein or a specific sugar chain linked to the marker protein, etc. can be used as the antibody specific for the marker protein. In the case of an intracellularly expressed marker protein which does not appear on the surface of cells (for example, a transcription factor or a subunit thereof), the marker protein of interest can be detected by expressing the marker protein with a reporter protein and detecting the reporter protein (for example, Non Patent Literature 4). This method may be preferably used when an appropriate cell surface marker is not found. The marker gene can be detected by use of a method of amplifying and/or detecting nucleic acid known in the art, for example, RT-PCR, microarray, biochip, or RNAseq.

The term "expression" is defined as transcription and/or translation of a certain nucleotide sequence driven by an intracellular promoter.

The term "positive" or "expressing" means that a protein or a gene is expressed in an amount detectable by an approach known in the art. The protein can be detected by use of immunological assay, for example, ELISA, immunostaining, or flow cytometry, using an antibody. In the case of an intracellularly expressed protein which does not appear on the surface of cells (for example, a transcription factor or a subunit thereof), the protein of interest can be detected by expressing the protein with a reporter protein and detecting the reporter protein. The gene can be detected by use of a method of amplifying and/or detecting nucleic acid, for example, RT-PCR, microarray, biochip, or RNAseq.

The term "negative" or "not expressed" means that the expression level of a protein or a gene is lower than the lower limit of detection in all or any of the known approaches as described above. The lower limit of detection for the expression of a protein or a gene may differ depending on each approach.

"Erythropoietin" (hereinafter, referred to as "EPO" in the present specification; "EPO" means erythropoietin protein unless otherwise specified) is a hematopoietic factor that promotes the production of erythrocytes and is composed of 165 amino acids. Its molecular weight is about 34000. EPO is secreted from hepatocytes in the fetal and neonatal periods and secreted from renal stromal cells in the later stages of pregnancy and adulthood (Non Patent Literature 1).

"CD73" is also called 5'-nucleotidase (5'-NT) and is a membrane protein encoded by NT5E gene. 70 kDa subunits form a dimer, which is in turn anchored to GPI and present on cell surface. CD73 is expressed mainly in mesenchymal stem cells, and its expression is also found in cells of the immune system such as T cells or B cells and in some epithelial cells and endothelial cells. CD73 has the enzymatic activity of 5'-nucleotidase and catalyzes the dephosphorylation of ribo- or deoxyribonucleoside monophosphate of purine and pyrimidine into the corresponding nucleoside.

"Desmin" is a protein that belongs to intermediate filament class III, a proteinous fiber system in the cytoplasm. Desmin is an essential factor for the structural integrity and survival of myocytes or the like.

"Vimentin" is a protein that belongs to intermediate filament class III, a proteinous fiber system in the cytoplasm, and is expressed in mesenchymal cells such as fibroblasts, vascular endothelial cells, smooth muscle cells, striated muscle cells, bone cells, chondrocytes, or neurilemma cells.

"Forkhead box D: FOXD1" is a reprogramming regulator and is a stage-specific marker whose expression is elevated in cells with advanced reprogramming. FOXD1 is not expressed in pluripotent stem cells.

"SOX10" is found to be expressed in all of neural crest cells. On the other hand, SOX10 is not expressed in renal stromal precursors or renal stromal cells.

"Nerve growth factor receptor (NGFR)" is a receptor to which a nerve growth factor (NGF) binds.

"HOXB4" is an Antp homeobox protein and is encoded by HOXB4 gene within a homeobox B gene cluster. HOXB4 protein has a homeobox DNA binding region and functions as a specific transcription factor during the development of an individual.

"Neural EGFL Like 2 (NELL2)" is a protein kinase C binding protein having epidermal growth factor (EGF)-like repeats. NELL2 is highly expressed in central nerves *in vivo* and also expressed in the cytoplasm of various stromal cells including renal stromal cells.

"Paired box protein-7 (PAX7)" is a transcription factor necessary for the development and differentiation of neural crest and forms a heterodimer with PAX3 (which is also necessary for the development and differentiation of neural crest), which in turn binds to DNA. PAX7 is expressed in muscle satellite cells and also in renal stromal cells *in vivo.*

"Nik related kinase (NRK)" is a serine/threonine kinase and functions to phosphorylate the TNF-JNK signaling pathway. The gene resides on the X chromosome and is expressed in reproductive organs such as the testicle and the ovary and also in renal stromal cells *in vivo.*

The term "comprise(s)" or "comprising" refers to inclusion of the element(s) following the term without limitations thereto. Thus, this suggests inclusion of the element(s) following the term but does not suggest exclusion of any other element.

The term "about" or "approximately" refers to a value which may vary up to plus or minus 30%, 25%, 20%, 15%, 10%, 8%, 6%, 5%, 4%, 3%, 2%, or 1% from the reference value. Preferably, the term "about" or "approximately" refers to a range from minus or plus 15%, 10%, 5%, or 1% from the reference value.

### Advantageous Effects of Invention

The present invention provides a technique for supplying renal stromal cells.

### Brief Description of Drawings

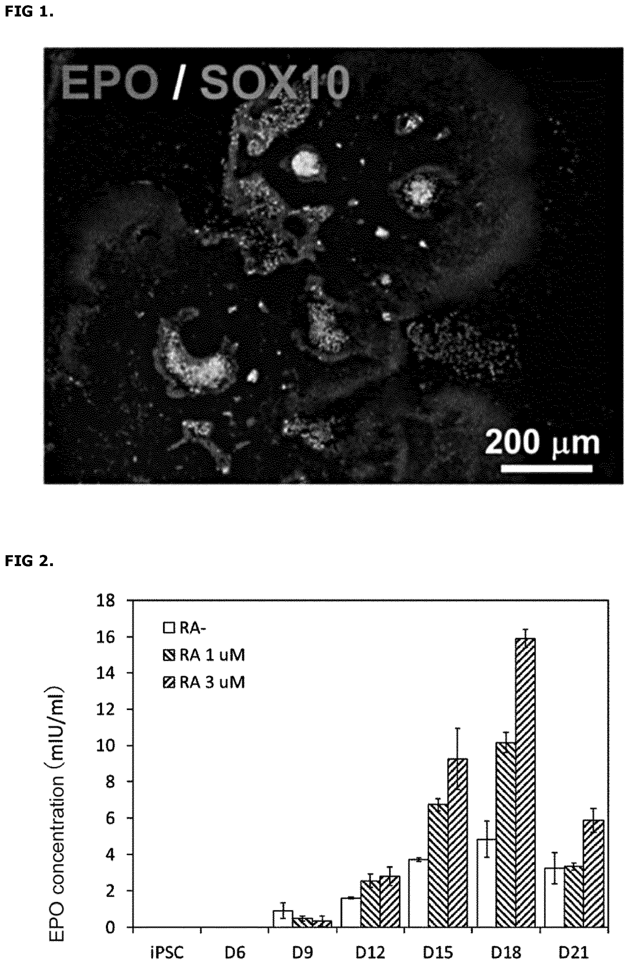

[Figure 1] Figure 1 is an immunohistochemical staining image showing the expression of EPO and SOX10 in neural crest cells induced from induced pluripotent stem cells.
[Figure 2] Figure 2 is a graph showing results of measuring the accumulation of EPO secreted into a medium from neural crest cells on each day of culture when a RA concentration was set to 0 µM (-), 1 µM (low) and 3 µM (high). The ordinate shows an EPO concentration (mIU/ml). The abscissa shows the days of culture (Days 6 to 21). "iPSC" depicts a measurement value in induced pluripotent stem cells as a negative control.

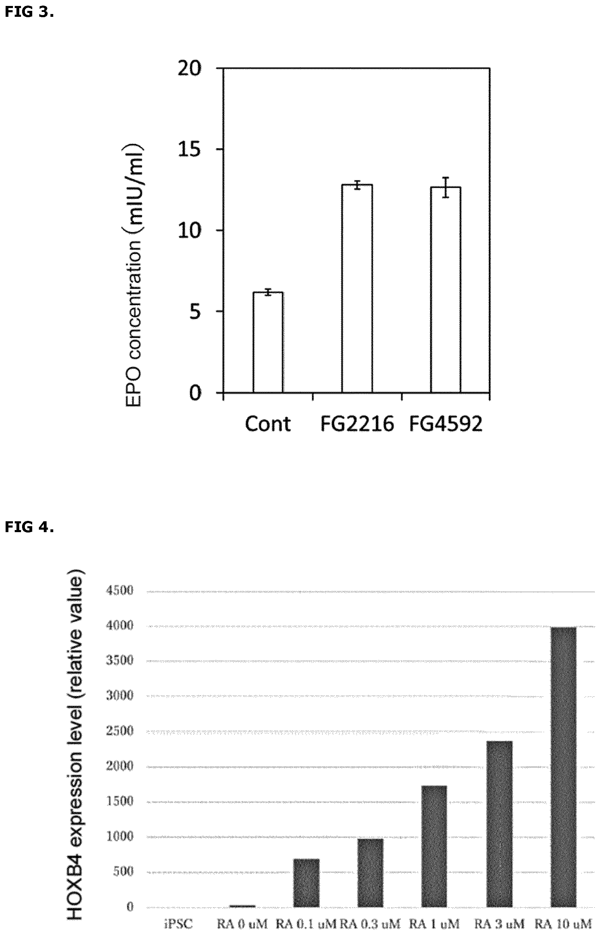

[Figure 3] Figure 3 is a graph showing results of measuring the amount of EPO secreted by the hypoxic stimulation (FG2216 or FG4592) of posteriorly shifted neural crest cells. The ordinate shows an EPO concentration (mIU/ml).
[Figure 4] Figure 4 is a graph showing results of measuring the expression level of HOXB4 in hindbrain neural crest cells induced from induced pluripotent stem cells. The ordinate shows the expression level of HOXB4 in the hindbrain neural crest cells by a relative value with the expression level in an induced pluripotent stem cell line ("iPSC") defined as 1. The abscissa shows the concentration of retinoic acid.

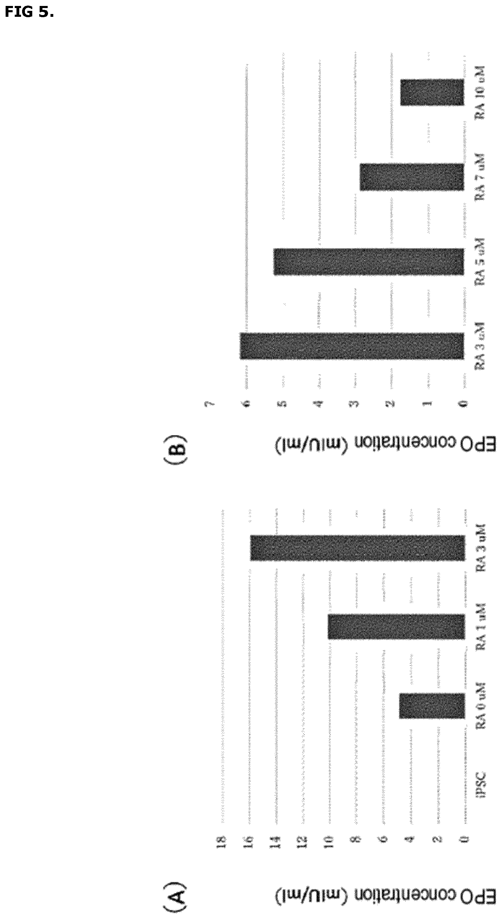

[Figure 5] Figure 5 is a graph showing results of measuring the accumulation of EPO secreted into a medium from hindbrain neural crest cells when a RA concentration was set to 0 to 10 µM. The ordinate shows an EPO concentration (mIU/ml). The abscissa shows the concentration of retinoic acid. "iPSC" depicts a measurement value in induced pluripotent stem cells as a negative control.

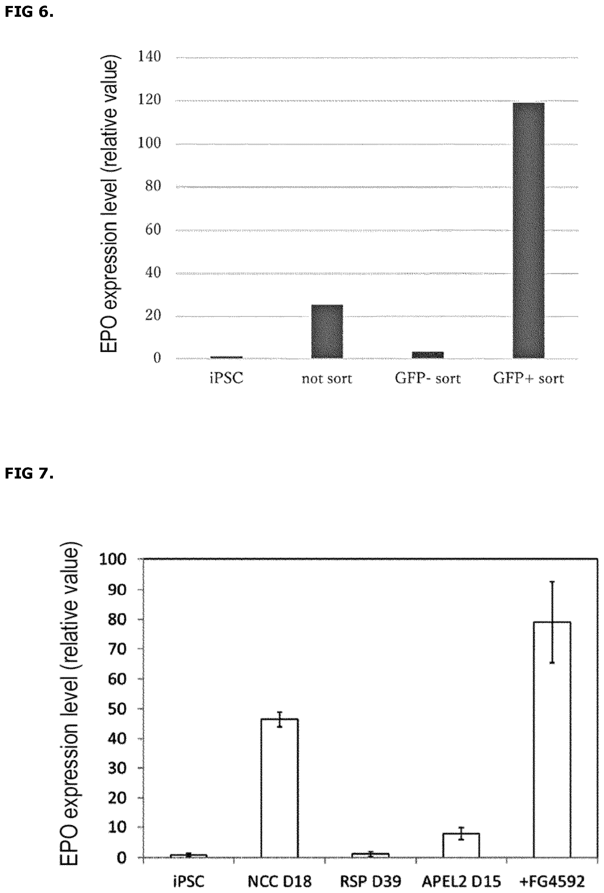

[Figure 6] Figure 6 is a graph showing results of measuring an EPO mRNA expression level in an EPO-emGFP knock-in human induced pluripotent stem cell line ("iPSC"), hindbrain neural crest cells before sorting ("not sort"), GFP-negative hindbrain neural crest cells ("GFP- sort") and GFP-positive hindbrain neural crest cells ("GFP+ sort"). The ordinate shows the EPO mRNA expression level by a relative value with the expression level in the EPO-emGFP knock-in human induced pluripotent stem cell line ("iPSC") defined as 1.
[Figure 7] Figure 7 is a graph showing results of measuring an EPO mRNA expression level in hindbrain neural crest cells ("NCC D18"), renal stromal precursors ("RSP D39"), renal stromal cells ("APEL D15") and hypoxically stimulated renal stromal cells ("+FG4592"). The ordinate shows the EPO mRNA expression level by a relative value with the expression level in the EPO-emGFP knock-in human induced pluripotent stem cell line ("iPSC") defined as 1.

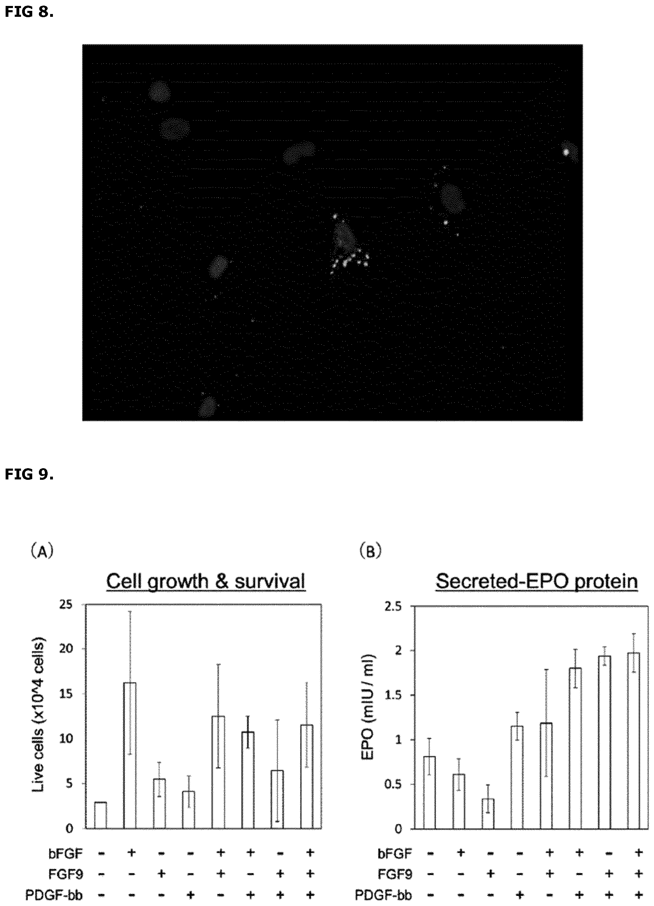

[Figure 8] Figure 8 is an immunohistochemical staining image showing the expression of hypoxia-responsive EPO protein in renal stromal cells induced from renal stromal precursors. The EPO protein is shown in red, and the cell nucleus is shown in blue.
[Figure 9] Figure 9 is a graph showing results of measuring the cell proliferation (A) and the amount of EPO secreted (B) of renal stromal cells induced in the presence of bFGF, FGF9 and PDGF-BB.

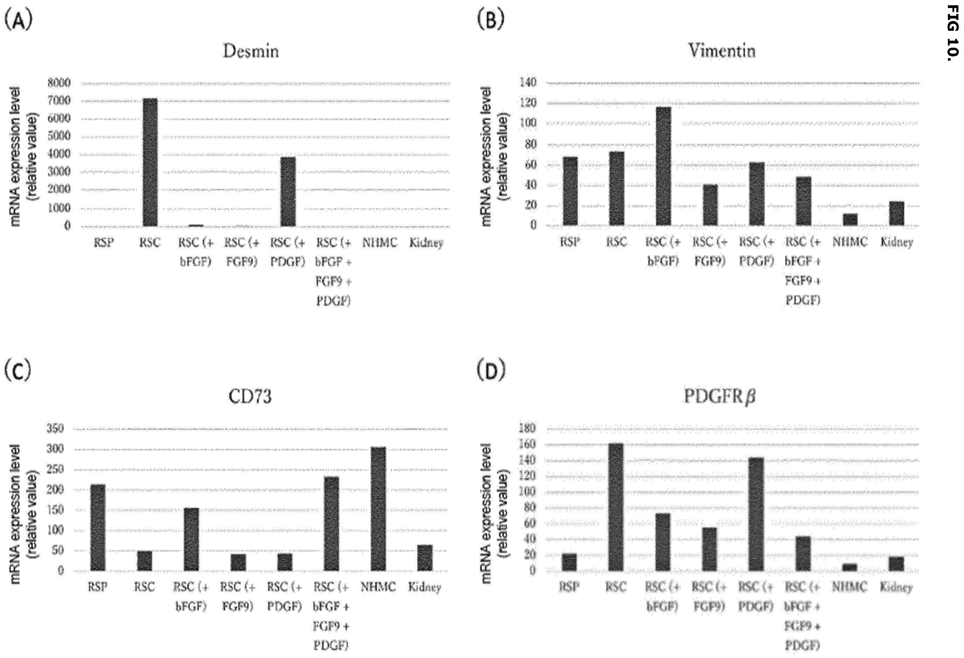

[Figure 10] Figure 10 is a graph showing results of measuring the expression levels of renal stromal cell markers (desmin, vimentin, CD73, and PDGFRβ) in hypoxically stimulated renal stromal cells. The ordinate shows a mRNA expression level by a relative value with the expression level in an induced pluripotent stem cell line defined as 1.

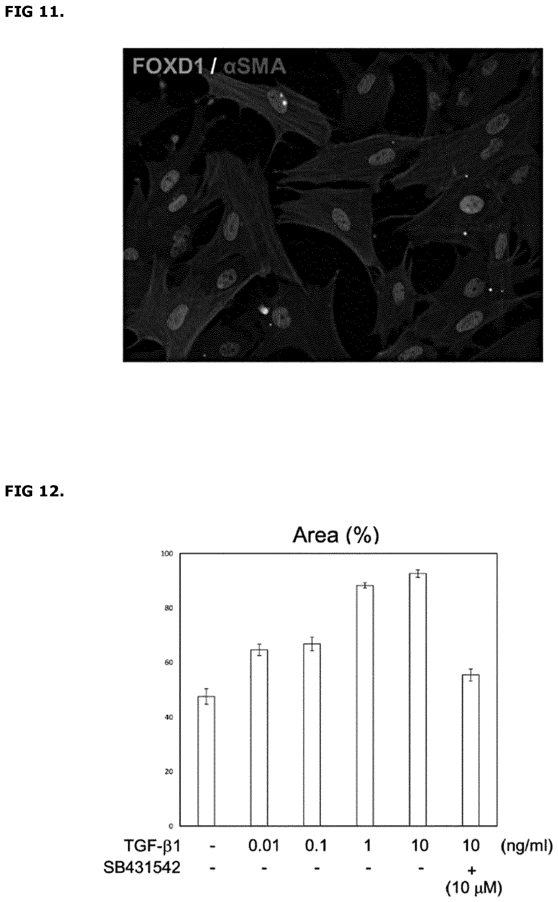

[Figure 11] Figure 11 is an immunohistochemical staining image showing the expression of αSMA and FOXD1 in myofibroblasts obtained by the differentiation of renal stromal cells by TGFβ1 stimulation.
[Figure 12] Figure 12 is a graph showing the proportion of myofibroblasts that were differentiated from renal stromal cells by TGFβ1-stimulation. The ordinate shows the proportion (%) of the area of tdTomato-stained cells to the total area of cultured cells.

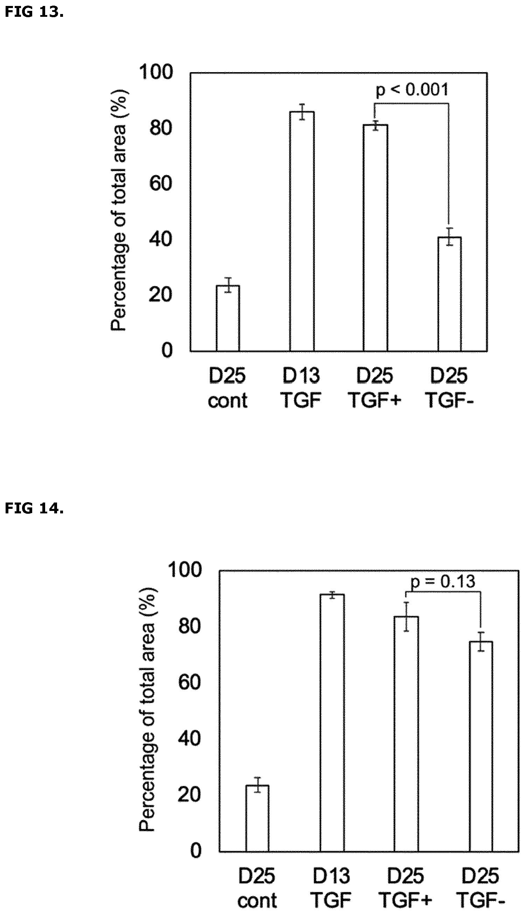

[Figure 13] In Figure 13, RSCs on day 4 of culture (D4) were treated with TGFβ1 (1 ng/ml) for 9 days (D13 TGF) and then further cultured in a TGFβ1-containing medium (D25 TGF+) or a TGFβ1-free medium (D25 TGF-) for 12 days. This figure shows the proportion (%) of the area of tdTomato-stained cells to the total area of cultured cells on D13 and D25. "D25 Cont" depicts a negative control (without TGFβ1 treatment).
[Figure 14] In Figure 14, RSCs on day 4 of culture (D4) were treated with TGFβ1 (10 ng/ml) for 9 days (D13 TGF) and then further cultured in a TGFβ1-containing medium (D25 TGF+) or a TGFβ1-free medium (D25 TGF-) for 12 days. This figure shows the proportion (%) of the area of tdTomato-stained cells to the total area of cultured cells on D13 and D25. "D25 Cont" depicts a negative control (without TGFβ1 treatment).

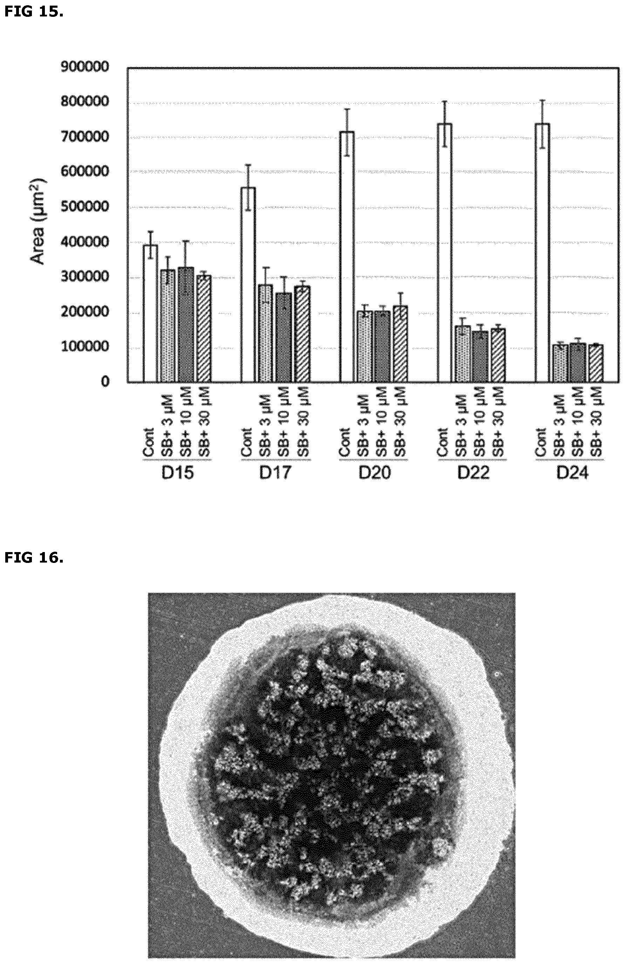

[Figure 15] In Figure 15, RSCs on day 4 of culture (D4) were treated with TGFβ1 (10 ng/ml) for 9 days (D13) and then further cultured in a TGFβ1-free medium (D15) for 2 days. SB431542 (3, 10 or 30 µM) was added to the medium of the cells on D15, and the cells were further cultured for 9 days (D24). This figure shows the area of tdTomato-stained cells on D15, D17, D20, D22 and D24. "Cont" depicts a negative control (without SB addition).
[Figure 16] Figure 16 is a microscope image of a kidney organoid.

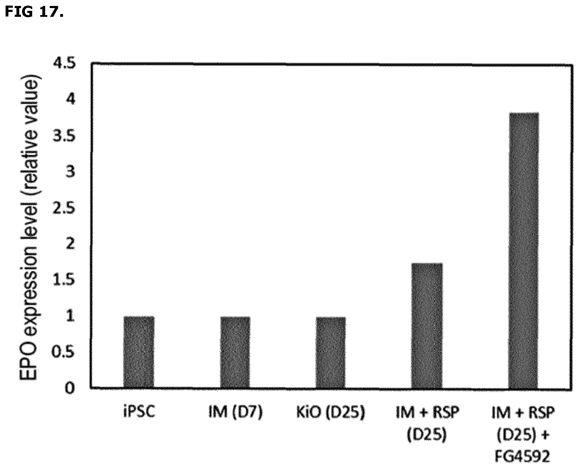

[Figure 17] Figure 17 is a graph showing results of measuring an EPO mRNA expression level in an induced pluripotent stem cell line ("iPSC"), an intermediate mesoderm ("IM (D7)"), a kidney organoid (Mini-kidney: "IM+RSP (D25)"), a kidney organoid containing no renal stromal precursor ("KiO (D25)") and a kidney organoid under hypoxic conditions (Mini-kidney: "IM+RSP (D25) + FG4592"). The ordinate shows the EPO mRNA expression level by a relative value with the expression level in the induced pluripotent stem cell line defined as 1.

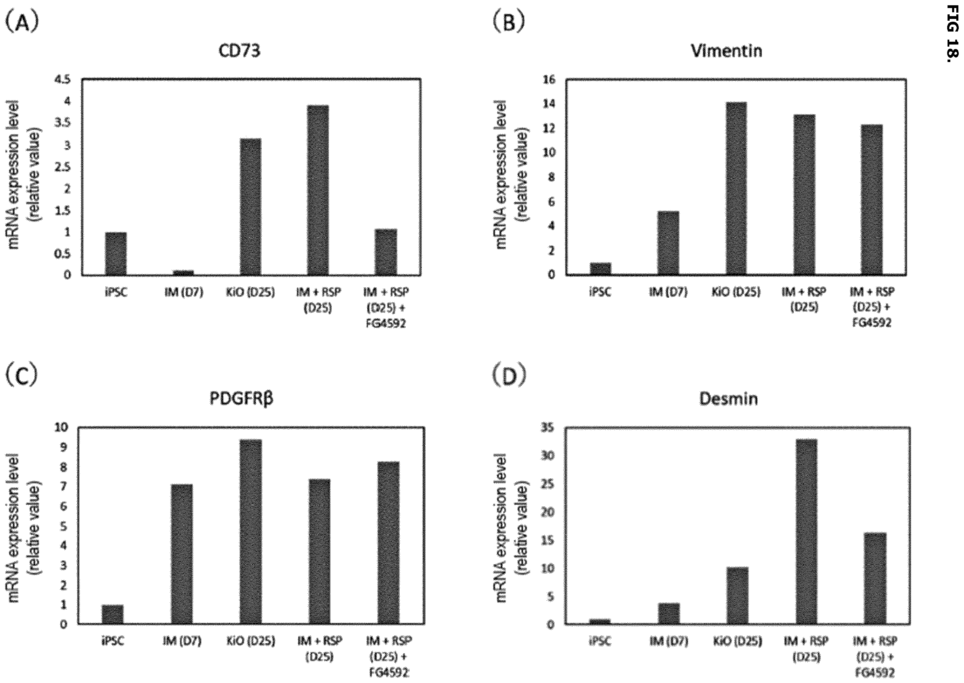

[Figure 18] Figure 18 is a graph showing results of measuring the expression levels of renal stromal cell markers (desmin, vimentin, CD73, and PDGFRβ) in an induced pluripotent stem cell line ("iPSC"), an intermediate mesoderm ("IM (D7)"), a kidney organoid (Mini-kidney: "IM+RSP (D25)"), a kidney organoid containing no renal stromal precursor ("KiO (D25)") and a kidney organoid under hypoxic conditions (Mini-kidney: "IM+RSP (D25) + FG4592"). The ordinate shows an mRNA expression level by a relative value with the expression level in the induced pluripotent stem cell line defined as 1.

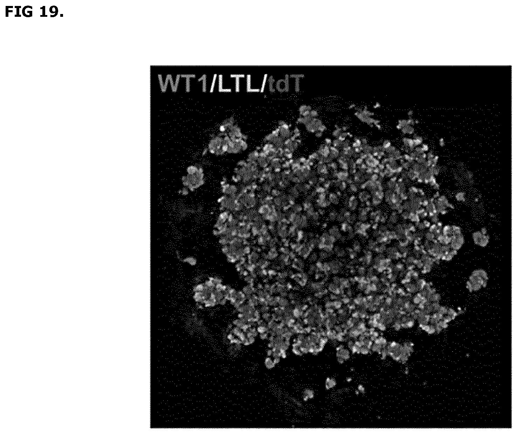

[Figure 19] Figure 19 is an immunohistochemical staining image showing the expression of a glomerular cell marker (WT1) and a renal tubular cell marker (LTL) in a kidney organoid (Mini-kidney).

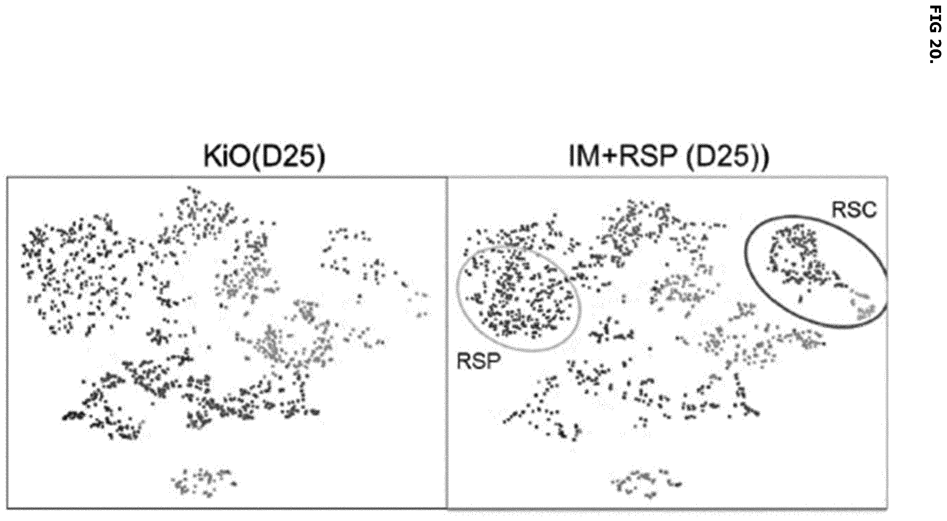

[Figure 20] Figure 20 is a T-SNE plot showing the cell populations of a kidney organoid containing RSPs (IM+RSP (D25); right) and a kidney organoid containing no RSP (KiO (D25); left).

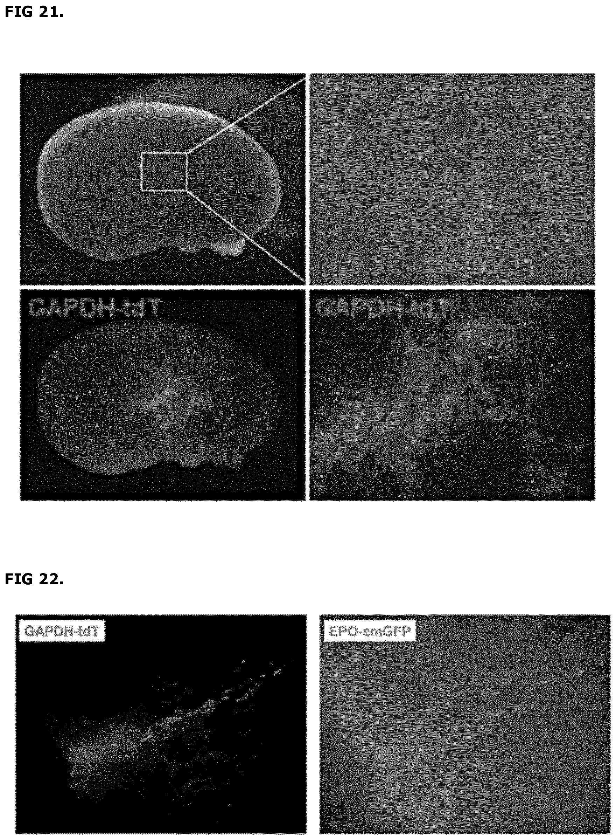

[Figure 21] Figure 21 shows fluorescent images taken under a stereoscopic microscope of the kidney excised after transplantation of a RSP cell mass derived from an EPO-emGFP/GAPDH-tdTomato (GAPDH-tdT) dual knock-in human iPS cell line into the kidney of an immunodeficient NOG mouse. Results of fluorescently observing GAPDH-tdT indicate that cells migrated from the transplanted cell mass and engrafted in the kidney.
[Figure 22] Figure 22 is fluorescent images of tissue sections containing a RSP (GAPDH-tdTomato-positive cell) engraftment site of the kidney excised after transplantation of a cell mass into the kidney, and shows that fluorescence ascribable to the expression of EPO-emGFP was detected in some cells of the transplanted RSPs.

### [Detailed Description of Invention]

Hereinafter, suitable modes for carrying out the present invention will be described. The embodiments described below are given merely for illustrating typical embodiments of the present invention. The scope of the present invention should not be interpreted as being limited by these embodiments.

### 1. Method for producing renal stromal cell

The method for producing renal stromal cells according to the present invention comprises the following step (3) and optionally comprises the following step (2) and/or step (1).
Step (3): the step of culturing renal stromal precursors in a medium comprising a platelet derived growth factor receptor agonist to obtain renal stromal cells.
Step (2): the step of inducing renal stromal precursors from neural crest cells.
Step (1): the step of culturing pluripotent stem cells in a medium comprising a GSK3β inhibitor, a TGFβ inhibitor, and retinoic acid and/or a derivative thereof to induce neural crest cells.

### 1-1. Step (3)

In this step, renal stromal precursors are cultured in a medium comprising a platelet derived growth factor receptor agonist to obtain renal stromal cells. The platelet derived growth factor receptor agonist has been found to have the activity of inducing the differentiation of renal stromal precursors into renal stromal cells and to function as a renal stromal cell inducer. The platelet derived growth factor receptor agonist is preferably a platelet derived growth factor (PDGF) .

The renal stromal precursors may be the ones obtained in the step (2), may be commercially obtained cells, or may be ex *vivo* cells separated from a living body.

In the case of using renal stromal precursors induced from induced pluripotent stem cells, renal stromal cells derived from induced pluripotent stem cells are produced in this step.

The medium (medium for use in producing renal stromal cells) comprises a platelet derived growth factor receptor agonist and preferably further comprises a basic fibroblast growth factor (bFGF) or fibroblast growth factor 9 (FGF9), more preferably bFGF and FGF9.

The basal medium is not particularly limited. For example, STEMdiff APEL2 medium (STEMCELL Technologies Inc., ST-05275), TeSR1 medium and Chemically Defined Medium (CDM) medium are suitably used. In addition, for example, BME medium, BGJb medium, CMRL 1066 medium, Glasgow MEM medium, improved MEM (IMEM) medium, improved MDM (IMDM) medium, Medium 199 medium, Eagle MEM medium, αMEM medium, DMEM medium (high glucose or low glucose), DMEM/F12 medium, Ham's medium, RPMI 1640 medium, Fischer's medium, and mixed media thereof may be used.

The CDM medium is not particularly limited. For example, a medium prepared from Iscove's modified Dulbecco's medium (manufactured by GE Healthcare Japan Corp.) may be used.

The basal medium may be supplemented with a substance for use in usual cell culture, such as Ham's F-12 nutrient mixture, albumin such as human serum albumin, polyvinyl alcohol (PVA), deionized BSA, linoleic acid, linolenic acid, cholesterol, insulin, apotransferrin, selenium, ethanolamine, monothioglycerol, Protein-free hybridoma mixture II (PFHMII), ascorbic acid, L-alanyl-L-glutamine and/or an antibiotic.

FGF and FGF9 derived from an appropriate animal species can be selected and used according to the animal species from which the renal stromal precursors to be cultured are derived. Preferably, factors derived from the same animal as the animal species from which the renal stromal precursors are derived are used.

PDGF is a growth factor that is involved in the regulation of migration and proliferation, etc. of mesenchymal cells such as fibroblasts. PDGF includes four types, PDGF-A, PDGF-B, PDGF-C and PDGF-D. Among them, the A chain and the B chain form a homo- or heterodimer through the formation of a disulfide bond, and thus the dimer has three isoforms, PDGF-AA, PDGF-AB and PDGF-BB. The C chain and the D chain each form a homodimer (PDGF-CC and PDGF-DD). In the present invention, PDGF-BB is preferred. PDGF-BB is not particularly limited, and, for example, #100-14B manufactured by PeproTech, Inc. can be used.

Full-length peptides or receptor binding fragments thereof can be used as PDGF, bFGF and FGF9.

Commercially available PDGF, bFGF and FGF9 can be obtained and used. bFGF and FGF9 are not particularly limited, and, for example, product numbers #068-04544 and #AF-100-23, respectively, from FUJIFILM Wako Pure Chemical Corp. can be used.

The concentration of PDGF to be added into the medium can be appropriately adjusted according to the subtype of the PDGF used and is, for example, 0.05 ng/ml to 1 µg/ml. The concentration is preferably 0.1 to 500 ng/ml, more preferably 1 to 100 ng/ml. In the case of using PDGF-BB, its concentration is, for example, 0.1 ng/ml to 1 µg/ml. The concentration is preferably 1 to 300 ng/ml, more preferably 3 to 100 ng/ml, particularly preferably about 10 ng/ml.

The concentration of bFGF to be added into the medium is, for example, 0.1 ng/ml to 1 µg/ml. The concentration is preferably 1 to 500 ng/ml, more preferably 10 to 300 ng/ml, particularly preferably about 40 ng/mL.

The concentration of FGF9 to be added into the medium is, for example, 0.1 ng/ml to 1 µg/ml. The concentration is preferably 1 to 500 ng/ml, more preferably 10 to 300 ng/ml, particularly preferably about 200 ng/mL.

In the case of using a platelet derived growth factor receptor agonist as an inducer for the differentiation of renal stromal precursors into renal stromal cells, the inducer may comprise the platelet derived growth factor receptor agonist as well as bFGF, FGF9, retinoic acid (RA), or the like.

The culture period in this step can be a period in which the renal stromal precursors differentiate into renal stromal cells. The culture period is not particularly limited and is, for example, 7 to 24 days, preferably 10 to 20 days, more preferably 12 to 18 days, particularly preferably about 15 days.

This step is preferably performed by adherent culture and may be performed by suspension culture.

For the adherent culture, a culture container, for example, a dish, a flask, a microplate, or a cell culture sheet such as OptiCell (product name) (Nunc), is used.

The container for use in adherent culture may be surface-treated in order to improve adhesiveness to cells (hydrophilicity), or coated with a substrate for cell adhesion such as collagen, gelatin, poly-L-lysine, poly-D-lysine, laminin, fibronectin, Matrigel, or vitronectin. A container without such surface treatment or coating is more preferably used.

In the suspension culture, the cells are dispersed into a medium, and an aggregated cell mass is formed while medium components and the internal oxygen concentration of the medium are uniformized by stirring or shaking. The suitable stirring rate is appropriately set according to a cell density and the size of a culture container. Excessive stirring or shaking places physical stress on the cells and inhibits aggregated cell mass formation. Thus, the stirring or shaking rate is controlled so as to be able to uniformize medium components and the internal oxygen concentration of the medium and so as not to inhibit aggregated cell mass formation. The suspension culture may be performed by still standing without stirring or shaking.

For the suspension culture, it is preferred to use a container with low-adhesion coating such as Prime surface (product name, Sumitomo Bakelite Co., Ltd.).

The culture temperature is not particularly limited and is 30 to 40°C (for example, 37°C). A carbon dioxide concentration in the culture container is on the order of, for example, 5%.

The production of renal stromal cells is confirmed by, for example, a method of measuring the expression of a marker protein or a marker gene. Provided that the obtained cells express EPO gene and/or protein, CD73 and PDGFRβ and preferably further express desmin and vimentin, the cells can be determined as renal stromal cells.

The marker protein can be detected by use of immunological assay using an antibody specific for the marker protein, for example, ELISA, immunostaining, or flow cytometry. The marker gene can be detected by use of a method of amplifying and/or detecting nucleic acid known in the art, for example, RT-PCR, microarray, or biochip.

The renal stromal cells obtained in this step possess a function of producing EPO, particularly, under hypoxic conditions (hypoxic response). The hypoxic response can be confirmed by treating cells with a compound that activate HIF signals, and detecting the elevated expression of EPO at the mRNA level or the protein level. For example, FG-2216 (Selleck Chemicals, #18382) and FG-4592 (Selleck Chemicals, #S1007) can be used as the compound that activates HIF signals.

The hypoxic response can also be confirmed by culturing cells of interest under a condition of a hypoxic concentration. The oxygen concentration under the condition of a hypoxic concentration can be an oxygen concentration in a hypoxic state that may occur in an *in vivo* environment. The oxygen concentration can be, for example, 20% or less, and can be 20 to 10%, 10 to 5%, or 5 to 1%.

Since there is a report stating that renal fibrosis is caused by the fibrosis of renal stromal cells having the ability to produce EPO, the renal stromal cells having the ability to produce EPO are useful as a material for the construction of renal fibrosis models that mimic a living body for the development of therapeutic drugs for renal fibrosis. Also, the renal stromal cells having the ability to produce EPO may be used in cell medicine for kidney damage aimed at treating or preventing, particularly, nephrogenic anemia.

The present invention also provides a frozen stock comprising the renal stromal cells obtained by this step.

The frozen stock can be produced by separating the obtained renal stromal cells from the medium by centrifugation, and suspending the separated cells in a cryopreservation solution for freezing. A conventional reagent for use in the cryopreservation of cells can be used as the cryopreservation solution. For example, Cryostem Freezing Medium (trade name) and CELLBANKER(R) are commercially available.

The frozen stock may be used, for example, for the preparation of a tissue model (kidney organoid) having renal stromal cells as a constituent.

### 1-2. Step (2)

In this step, renal stromal precursors are induced from neural crest cells.

The present invention provides a method for producing renal stromal precursors from neural crest cells.

The neural crest cells may be the ones obtained in the step (1), may be commercially obtained cells, or may be *ex vivo* cells separated from a living body.

Examples of the commercially available neural crest cells include Human Hair Follicle Outer Root Sheath Cells (manufactured by Cosmo Bio Co., Ltd.) and 09-1 Mouse Cranial Neural Crest Cell Line (manufactured by Merck Millipore).

Neural crest cells reportedly exist, for example, in human embryonic neural tube approximately 30 days after fertilization, mouse embryonic neural tube approximately the 9th fetal day, and human, swine and rodent adult skin (Betters et al., Developmental biology, 2010, 344(2): 578-592, Jiang et al., Development, 2000, 127(8): 1607-1616, Dupin et al., Developmental biology, 2012, 366(1): 83-95, Nagoshi et al., Cell Stem Cell 2, April 2008, 392-403). Such neural crest cells may be collected by use of a known method (for example, Motohashi et al., Biology open, 2016, 5:311-322, Pfaltzgraffet al., Journal of Visualized Experiments, 2012, 64:4134), appropriately posteriorly shifted, and then subjected to this step.

Hindbrain neural crest cells (hindbrain NCCs: hNCCs) are preferably used as the neural crest cells.

In the case of using neural crest cells induced from induced pluripotent stem cells, renal stromal precursors derived from induced pluripotent stem cells are produced in this step.

The neural crest cells are cultured in a medium for mesenchymal stromal cell induction to obtain renal stromal precursors. For example, StemPro MSC xenofree medium (Thermo Fisher Scientific, Inc.: A1067501) can be used as the medium for mesenchymal stromal cell induction.

The culture period in this step can be a period in which the neural crest cells differentiate into renal stromal precursors. The culture period is not particularly limited and is, for example, 10 to 90 days, preferably 20 to 50 days, more preferably 30 to 40 days, particularly preferably 30 to 35 days.

This step is preferably performed by adherent culture and may be performed by suspension culture.

For the adherent culture, a culture container, for example, a dish, a flask, a microplate, or a cell culture sheet such as OptiCell (product name) (Nunc), is used.

The container for use in adherent culture may be surface-treated in order to improve adhesiveness to cells (hydrophilicity), or coated with a substrate for cell adhesion such as collagen, gelatin, poly-L-lysine, poly-D-lysine, laminin, fibronectin, Matrigel, or vitronectin. A container without such surface treatment or coating is more preferably used.

In the suspension culture, the cells are dispersed into a medium, and an aggregated cell mass is formed while medium components and the internal oxygen concentration of the medium are uniformized by stirring or shaking. The suitable stirring rate is appropriately set according to a cell density and the size of a culture container. Excessive stirring or shaking places physical stress on the cells and inhibits aggregated cell mass formation. Thus, the stirring or shaking rate is controlled so as to be able to uniformize medium components and the internal oxygen concentration of the medium and so as not to inhibit aggregated cell mass formation. The suspension culture may be performed by still standing without stirring or shaking.

For the suspension culture, it is preferred to use a container with low-adhesion coating such as Prime surface (product name, Sumitomo Bakelite Co., Ltd.).

The culture temperature is not particularly limited and is 30 to 40°C (for example, 37°C). A carbon dioxide concentration in the culture container is on the order of, for example, 5%.

Hereinafter, the aggregated cell mass is also simply referred to as "cell mass" in the present specification.

The production of renal stromal precursors may be confirmed, for example, by confirming the ability to induce the differentiation of the cells into renal stromal cells.

Also, the production of renal stromal precursors is confirmed by, for example, a method of measuring the expression of a marker protein or a marker gene. Provided that the obtained cells express FOXD1, CD73 and PDGFRβ and do not express erythropoietin, the cells can be determined as renal stromal precursors.

The present invention also provides a frozen stock comprising the renal stromal precursors obtained by this step.

The frozen stock can be produced by separating the obtained renal stromal precursors from the medium by centrifugation, and suspending the separated cells in a cryopreservation solution for freezing. A conventional reagent for use in the cryopreservation of cells can be used as the cryopreservation solution. For example, Cryostem Freezing Medium (trade name) and CELLBANKER(R) are commercially available.

The frozen stock may be used, for example, as a starting material for obtaining renal stromal cells from renal stromal precursors. The frozen stock may be used for the preparation of a tissue model (kidney organoid) having renal stromal precursors as a constituent.

### 1-3. Step (1)

In this step, pluripotent stem cells (particularly, induced pluripotent stem cells) are cultured in a medium comprising a GSK3β inhibitor, a TGFβ inhibitor, and retinoic acid and/or a derivative thereof to induce neural crest cells (preferably hindbrain neural crest cells).

The differentiation of pluripotent stem cells into various neural crest cells can be induced according to a known method described in a literature (for example, Non Patent Literatures 3 and 4). In the case of inducing the differentiation of human induced pluripotent stem cells into neural crest cells, the induced pluripotent stem cells are inoculated to a dish or the like, adherent-cultured, then adherent-cultured in a medium comprising a TGFβ inhibitor and a GSK3β inhibitor, and then adherent-cultured in a medium further supplemented with retinoic acid and/or a derivative thereof, and thereby allowed to differentiate into neural crest cells.

The term "pluripotent stem cells" that may be used in the present invention refer to stem cells that can differentiate into tissues and cells having various different shapes and functions of a living body and have the ability to differentiate into cells of any lineage of the 3 germ layers (endoderm, mesoderm, and ectoderm). Examples thereof include, but are not particularly limited to, embryonic stem cells (ESCs), embryonic stem cells derived from cloned embryos obtained by nuclear transplantation, spermatogonial stem cells, embryonic germ cells, and induced pluripotent stem cells (herein also referred to as "iPSCs"). The term "multipotent stem cells" that may be used in the present invention refer to stem cells having the ability to differentiate into plural and limited numbers of linages of cells. Examples of the "multipotent stem cells" that may be used in the present invention include dental pulp stem cells, oral mucosa-derived stem cells, hair follicle stem cells, and somatic stem cells derived from cultured fibroblasts or bone marrow stem cells. The pluripotent stem cells are preferably ESCs and iPSCs.

Available "ESCs" include murine ESCs such as various murine ESC lines established by inGenious Targeting Laboratory, Riken (Institute of Physical and Chemical Research), and the like, and human ESCs such as various human ESC lines established by University of Wisconsin, NIH, Riken, Kyoto University, National Center for Child Health and Development, Cellartis, and the like. For example, CHB-1 to CHB-12 lines, RUES1 line, RUES2 line, and HUES1 to HUES28 lines distributed by ESI Bio, H1 line and H9 line distributed by WiCell Research, and KhES-1 line, KhES-2 line, KhES-3 line, KhES-4 line, KhES-5 line, SSES1 line, SSES2 line, and SSES3 line distributed by Riken can be used as the human ESC lines.

The term "induced pluripotent stem cells" refer to cells that are obtained by reprograming mammalian somatic cells or undifferentiated stem cells by introducing particular factors (nuclear reprogramming factors). At present, there are various "induced pluripotent stem cells" and iPSCs established by Yamanaka, et al. by introducing the 4 factors Oct3/4, Sox2, Klf4, c-Myc into murine fibroblasts (Takahashi K, Yamanaka S., Cell, (2006) 126: 663-676); iPSCs derived from human cells, established by introducing similar 4 factors into human fibroblasts (Takahashi K, Yamanaka S., et al. Cell, (2007) 131: 861-872.); Nanog-iPSCs established by sorting cells using expression of Nanog as an indicator after introduction of the 4 factors (Okita, K., Ichisaka, T., and Yamanaka, S. (2007). Nature 448, 313-317.); iPSCs produced by a method not using c-Myc (Nakagawa M, Yamanaka S., et al. Nature Biotechnology, (2008) 26, 101-106); iPSCs established by introducing 6 factors by a virus-free method (Okita K et al. Nat. Methods 2011 May; 8(5): 409-12, Okita K et al. Stem Cells. 31 (3) 458-66); and the like may be also used. Also, induced pluripotent stem cells established by introducing the 4 factors OCT3/4, SOX2, NANOG, and LIN28 by Thomson et al. (Yu J., Thomson JA. et al., Science (2007) 318: 1917-1920.); induced pluripotent stem cells produced by Daley et al. (Park IH, Daley GQ.et al., Nature (2007) 451: 141-146); induced pluripotent stem cells produced by Sakurada et al. (Japanese Unexamined Patent Application Publication No. 2008-307007) and the like may be used.

In addition, any of known induced pluripotent stem cells known in the art described in all published articles (for example, Shi Y., Ding S., et al., Cell Stem Cell, (2008) Vol3, Issue 5,568-574;, Kim JB., Scholer HR., et al., Nature, (2008) 454, 646-650; Huangfu D., Melton, DA., et al., Nature Biotechnology, (2008) 26, No 7, 795-797) or patents (for example, Japanese Unexamined Patent Application Publication No. 2008-307007, Japanese Unexamined Patent Application Publication No. 2008-283972, US2008-2336610, US2009-047263, WO2007-069666, WO2008-118220, WO2008-124133, WO2008-151058, WO2009-006930, WO2009-006997, WO2009-007852) can be used.

Available induced pluripotent cell lines include various iPSC lines established by NIH, Riken, Kyoto University and the like. Examples of such human iPSC lines include HiPS-RIKEN-1A line, HiPS-RIKEN-2A line, HiPS-RIKEN-12A line, and Nips-B2 line from Riken, and 253G1 line, 201B7 line, 409B2 line, 454E2 line, 606A1 line, 610B1 line, 648A1 line, 1231A3 line, Ff- I14s04 line, and QHJ I14s04 line from Kyoto University. 1231A3 line is preferred.

The medium comprises a GSK3β inhibitor, a TGFβ inhibitor, and retinoic acid and/or a derivative thereof. Retinol, retinal, tretinoin, isotretinoin, alitretinoin, etretinate, acitretin, tazarotene, bexarotene, or adapalene may be used as the derivative of retinoic acid. Two or more of these derivatives may be used in combination. Hereinafter, "retinoic acid and/or a derivative thereof" is also simply referred to as "retinoic acid, etc."

The basal medium is not particularly limited. For example, a mixture of solutions A, B and C of StemFit AK03, TeSR1 medium and Chemically Defined Medium (CDM) medium are suitably used. In addition, for example, BME medium, BGJb medium, CMRL 1066 medium, Glasgow MEM medium, improved MEM (IMEM) medium, improved MDM (IMDM) medium, Medium 199 medium, Eagle MEM medium, αMEM medium, DMEM medium (high glucose or low glucose), DMEM/F12 medium, Ham's medium, RPMI 1640 medium, Fischer's medium, and mixed media thereof may be used.

The CDM medium is not particularly limited. For example, a medium prepared from Iscove's modified Dulbecco's medium (manufactured by GE Healthcare Japan Corp.) can be used.

The basal medium may be supplemented with a substance for use in usual cell culture, such as apotransferrin, monothioglycerol, bovine serum albumin (BSA), insulin and/or an antibiotic.

Examples of the TGFβ inhibitor include SB431542 (4-[4-(1,3-benzodioxol-5-yl)-5-(2-pyridinyl)-1H-imidazol-2-yl]-benzamide), A83-01(3-(6-methyl-2-pyridinyl)-n-phenyl-4-(4-quinolinyl)-1h-pyrazole-1-carbothioamide), LDN193189 (4-[6-[4-(1-Piperazinyl)phenyl]pyrazolo[1,5-a]pyrimidin-3-yl]-quinoline), Wnt3a/BIO (Wnt Family Member 3A/ (2Z, 3E)-6'-Bromo-3-(hydroxyimino)-[2,3'-biindolinylidene]-2'-one), BMP4(Bone morphogenetic protein 4), GW788388 (4-[4-[3-(2-pyridinyl)-1h-pyrazol-4-yl]-2-pyridinyl]-n-(tetrahydro-2h-pyran-4-yl)-benzamide), SM16 (4-[4-(1,3-Benzodioxol-5-yl)-5-(6-methyl-2-pyridinyl)-1H-imidazol-2-yl]-bicyclo[2.2.2]octane-1-carboxamide), IN-1130 (3-[[5-(6-Methyl-2-pyridinyl)-4-(6-quinoxalinyl)-1H-imidazol-2-yl]methyl]-benzamide), GW6604 (2-Phenyl-4-[3-(pyridin-2-yl)-1H-pyrazol-4-yl]pyridine) and SB505124 (2-[4-(1,3-benzodioxol-5-yl)-2-(1,1-dimethylethyl)-1h-imidazol-5-yl]-6-methyl-pyridine). Two or more of these TGFβ inhibitors may be used in combination.

The concentration of the TGFβ inhibitor to be added into the medium is appropriately adjusted according to the type of the TGFβ inhibitor to be added, and is, for example, 0.1 to 50 µM, preferably 1 to 20 µM.

In the case of using SB431542 (4-[4-(1,3-benzodioxol-5-yl)-5-(2-pyridinyl)-1H-imidazol-2-yl]-benzamide), its concentration to be added can be, for example, 1 to 100 µM, preferably 5 to 20 µM, particularly preferably about 10 µM.

Examples of the GSK3β inhibitor include CHIR98014 (N6-[2-[[4-(2,4-dichlorophenyl)-5-(1H-imidazol-2-yl)-2-pyrimidinyl]amino]ethyl]-3-nitro-2,6-pyridinediamine), CHIR99021 (6-{2-[4-(2,4-Dichloro-phenyl)-5-(5-methyl-1H-imidazol-2-yl)-pyrimidin-2-ylamino]-ethylamino}-nicotinonitrile), CP21R7 (CP21R7), LY2090314 (3-[9-Fluoro-1,2,3,4-tetrahydro-2-(1-piperidinylcarbonyl)pyrrolo[3,2,1-jk][1,4]benzodiazepin-7-yl]-4-imidazo[1,2-a]pyridin-3-yl-1h-pyrrole-2,5-dione), TDZD-8 (2-methyl-4-(phenylmethyl)-1,2,4-thiadiazolidine-3,5-dione), SB216763 (3-(2,4-Dichlorophenyl)-4-(1-methyl-1H-indol-3-yl)-1H-pyrrole-2,5-dione), TWS-119 (3-[[6-(3-Aminophenyl)-7H-pyrrolo[2,3-d]pyrimidin-4-yl]oxy]phenol), kenpaullone, 1-azakenpaullone, SB415286 ([3-[(3-chloro-4-hydroxyphenyl)amino]-4-(2-nitrophenyl)-1Hpyrrole-2,5-dione]), AR-AO144-18 (1-[(4-methoxyphenyl)methyl]-3-(5-nitro-1,3-thiazol-2-yl)urea), CT99021, CT20026, BIO ((2'Z,3'E)-6-bromoindirubin-3'-oxime), BIO-acetoxime, pyridocarbazole-cyclopentadienyl ruthenium complexes, OTDZT, alpha-4-dibromoacetophenone, and lithium. Two or more of these GSK3β inhibitors may be used in combination.

The GSK3β inhibitor is not limited to these substances. For example, an antisense oligonucleotide or siRNA against GSK3β mRNA, an antibody that binds to GSK3β, or a dominant negative GSK3β mutant can also be used as the GSK3β inhibitor. These GSK3β inhibitors are commercially available or can be synthesized according to a known method.

The concentration of the GSK3β inhibitor to be added into the medium is appropriately adjusted according to the type of the GSK3β inhibitor to be added, and is, for example, 0.1 to 10 µM, preferably 0.5 to 2 µM.

In the case of using CHIR99021, its concentration to be added can be, for example, 0.1 to 10 µM, preferably 0.5 to 2 µM, particularly preferably about 1 µM.

The concentration of the retinoic acid and/or the derivative thereof to be added into the medium is appropriately adjusted according to the type of the compound to be added, and is, for example, 0.001 to 50 µM, preferably 0.1 to 10 µM.

In the case of inducing hindbrain neural crest cells (hNCCs) using retinoic acid, its concentration to be added can be, for example, 0.1 to 10 µM, preferably 1 to 5 µM, particularly preferably about 3 µM.

The culture period in the medium comprising the TGFβ inhibitor and the GSK3β inhibitor is, for example, 0 to 24 days, particularly, about 18 days. The culture period in the medium further supplemented with the retinoic acid, etc. is, for example, 3 to 24 days, particularly, about 12 days.

This step is preferably performed by adherent culture and may be performed by suspension culture.

For the adherent culture, a culture container, for example, a dish, a flask, a microplate, or a cell culture sheet such as OptiCell (product name) (Nunc), is used.

The container for use in adherent culture may be surface-treated in order to improve adhesiveness to cells (hydrophilicity), or coated with a substrate for cell adhesion such as collagen, gelatin, poly-L-lysine, poly-D-lysine, laminin, fibronectin, Matrigel, or vitronectin. A container without such surface treatment or coating is more preferably used.

In the suspension culture, the cells are dispersed into a medium, and an aggregated cell mass is formed while medium components and the internal oxygen concentration of the medium are uniformized by stirring or shaking. The suitable stirring rate is appropriately set according to a cell density and the size of a culture container. Excessive stirring or shaking places physical stress on the cells and inhibits aggregated cell mass formation. Thus, the stirring or shaking rate is controlled so as to be able to uniformize medium components and the internal oxygen concentration of the medium and so as not to inhibit aggregated cell mass formation. The suspension culture may be performed by still standing without stirring or shaking.

For the suspension culture, it is preferred to use a container with low-adhesion coating such as Prime surface (product name, Sumitomo Bakelite Co., Ltd.).

The culture temperature is not particularly limited and is 30 to 40°C (for example, 37°C). A carbon dioxide concentration in the culture container is on the order of, for example, 5%.

The neural crest cells obtained in this step can be separated by a known approach, for example, magnetic activated cell sorting (FACS) or magnetic cell sorting (MACS). The neural crest cells in a cell population can be enriched by separating cells positive to the expression of a predetermined surface marker (for example, CD271, CD49D and HNK-1) from the cell population comprising the neural crest cells.

In this step, the obtained neural crest cells and/or the enriched neural crest cells may be maintenance-cultured and/or expansion-cultured for a given period (hereinafter, also referred to as "step (1B)" in the present specification) before being subjected to the step (2) .

### 1-4. Step (1B)

The neural crest cells can be maintained and expanded by suspension culture in a medium comprising a GSK3β inhibitor, a TGFβ inhibitor, EGF and bFGF.

The basal medium is not particularly limited. For example, a mixture of solutions A and B of StemFit AK03, TeSR1 medium and Chemically Defined Medium (CDM) medium are suitably used. In addition, for example, BME medium, BGJb medium, CMRL 1066 medium, Glasgow MEM medium, improved MEM (IMEM) medium, improved MDM (IMDM) medium, Medium 199 medium, Eagle MEM medium, αMEM medium, DMEM medium (high glucose or low glucose), DMEM/F12 medium, Ham's medium, RPMI 1640 medium, Fischer's medium, and mixed media thereof may be used.

The CDM medium is not particularly limited. For example, a medium prepared from Iscove's modified Dulbecco's medium (manufactured by GE Healthcare Japan Corp.) can be used.

The basal medium may be supplemented with a substance for use in usual cell culture, such as apotransferrin, monothioglycerol, bovine serum albumin (BSA), insulin and/or an antibiotic.

The concentration of the TGFβ inhibitor to be added into the medium is appropriately adjusted according to the type of the TGFβ inhibitor to be added, and is, for example, 0.1 to 50 µM, preferably 1 to 20 µM.

In the case of using SB431542 (4-[4-(1,3-benzodioxol-5-yl)-5-(2-pyridinyl)-1H-imidazol-2-yl]-benzamide), its concentration to be added can be, for example, 1 to 100 µM, preferably 5 to 20 µM, particularly preferably about 10 µM.

The concentration of the GSK3β inhibitor to be added into the medium is appropriately adjusted according to the type of the GSK3β inhibitor to be added, and is, for example, 0.1 to 10 µM, preferably 0.5 to 5 µM.

In the case of using CHIR99021, its concentration to be added can be, for example, 0.1 to 10 µM, preferably 0.5 to 5 µM, particularly preferably about 3 µM.

The concentration of bFGF to be added is not particularly limited and is, for example, 10 to 200 ng/ml, preferably 20 to 40 ng/ml.

The concentration of EGF to be added is not particularly limited and is, for example, 5 to 100 ng/ml, preferably 20 to 40 ng/ml.

In the suspension culture, the neural crest cells are dispersed into a medium, and an aggregated cell mass is formed while medium components and the internal oxygen concentration of the medium are uniformized by stirring or shaking. The suitable stirring rate is appropriately set according to a cell density and the size of a culture container. Excessive stirring or shaking places physical stress on the cells and inhibits aggregated cell mass formation. Thus, the stirring or shaking rate is controlled so as to be able to uniformize medium components and the internal oxygen concentration of the medium and so as not to inhibit aggregated cell mass formation. The suspension culture may be performed by still standing without stirring or shaking.

The culture temperature is not particularly limited and is 30 to 40°C (for example, 37°C). A carbon dioxide concentration in the culture container is on the order of, for example, 5%.

The culture period in this step can be a period in which a cell mass is formed. The culture period is appropriately adjusted. The culture period is preferably 24 hours to 7 days, more preferably 3 to 5 days, particularly preferably about 3 days.

In the meantime, the cells may be appropriately passaged. The passage is performed, for example, every 5 to 8 days after inoculation. The passage intervals are a period sufficient for the expansion of an aggregated cell mass and are preferably a period shorter than a period in which the aggregated cell mass becomes too large to allow oxygen or nutrients to reach cells inside the aggregated cell mass.

### 2. Method for producing kidney organoid

The method for producing a kidney organoid according to the present invention comprises the step of coculturing renal stromal cells or renal stromal precursors, and an intermediate mesoderm.

The renal stromal cells can be the ones obtained in the step (3) and may be commercially obtained cells, or may be ex *vivo* cells separated from a living body.

The renal stromal precursors can be the ones obtained in the step (2) and may be commercially obtained cells, or may be *ex vivo* cells separated from a living body.

The intermediate mesoderm may be induced from pluripotent stem cells (for example, induced pluripotent stem cells).

The induction of the intermediate mesoderm from pluripotent stem cells and the induction of the kidney organoid from the intermediate mesoderm and the renal stromal cells or the renal stromal precursors can be performed on the basis of known approaches described in literatures (Non Patent Literatures 6 and 7).

Specifically, human induced pluripotent stem cells are first cultured in a medium comprising a GSK3β inhibitor and then cultured in a medium comprising FGF9 and heparin to obtain an intermediate mesoderm.

The concentration of the GSK3β inhibitor to be added into the medium is, for example, 0.1 to 10 µM, preferably 1 to 10 µM, more preferably 3 to 9 µM, particularly preferably about 8 µM.

The concentration of FGF9 to be added into the medium is, for example, 0.1 ng/ml to 1 µg/ml, preferably 1 to 500 ng/ml, more preferably 10 to 300 ng/ml, particularly preferably about 200 ng/ml.

The concentration of heparin to be added into the medium is, for example, 0.01 to 100 µg/ml, preferably 0.1 to 10 µg/ml, particularly preferably about 1 µg/ml.

The culture period in the medium comprising the GSK3β inhibitor is, for example, 2 to 6 days, particularly, about 4 days.

The culture period in the medium comprising FGF9 and heparin is, for example, 2 to 6 days, particularly, about 3 days.

Next, the intermediate mesoderm is dispersed and mixed with renal stromal cells or renal stromal precursors to prepare a cell mass (pellet).

The mixing ratio between the cells dispersed from the intermediate mesoderm and the renal stromal cells or the renal stromal precursors is, for example, 1 to 0.002, preferably 0.02 to 0.6, more preferably 0.2 to 0.4, of the latter with respect to 1 of the former.

The cell mass (pellet) is treated with a medium comprising a GSK3β inhibitor and heparin, then cultured in a medium comprising FGF9 and heparin, and further cultured in a medium comprising heparin to obtain a kidney organoid.

The concentration of the GSK3β inhibitor to be added into the medium is, for example, 0.1 to 10 µM, preferably 1 to 10 µM, more preferably 3 to 7 µM, particularly preferably about 5 µM.

The concentration of FGF9 to be added into the medium is, for example, 0.1 ng/ml to 1 µg/ml, preferably 1 to 500 ng/ml, more preferably 10 to 300 ng/ml, particularly preferably about 200 ng/ml.

The concentration of heparin to be added into the medium is, for example, 0.01 to 100 µg/ml, preferably 0.1 to 10 µg/ml, particularly preferably about 1 µg/ml.

The treatment time in the medium comprising the GSK3β inhibitor and heparin is, for example, 0.5 to 2 hours, particularly, about 1 hour.

The culture period in the medium comprising FGF9 and heparin is, for example, 3 to 7 days, particularly, about 5 days.

The culture period in the medium comprising heparin is, for example, 7 to 30 days, particularly, about 21 days.

The basal medium is not particularly limited. For example, STEMdiff APEL2 medium (STEMCELL Technologies Inc., ST-05275), TeSR1 medium and Chemically Defined Medium (CDM) medium are suitably used. In addition, for example, BME medium, BGJb medium, CMRL 1066 medium, Glasgow MEM medium, improved MEM (IMEM) medium, improved MDM (IMDM) medium, Medium 199 medium, Eagle MEM medium, αMEM medium, DMEM medium (high glucose or low glucose), DMEM/F12 medium, Ham's medium, RPMI 1640 medium, Fischer's medium, and mixed media thereof may be used.

The CDM medium is not particularly limited. For example, a medium prepared from Iscove's modified Dulbecco's medium (manufactured by GE Healthcare Japan Corp.) may be used.

The basal medium may be supplemented with a substance for use in usual cell culture, such as Ham's F-12 nutrient mixture, albumin such as human serum albumin, polyvinyl alcohol (PVA), deionized BSA, linoleic acid, linolenic acid, cholesterol, insulin, apotransferrin, selenium, ethanolamine, monothioglycerol, Protein-free hybridoma mixture II (PFHMII), ascorbic acid, L-alanyl-L-glutamine and/or an antibiotic.

This step is preferably performed by adherent culture and may be performed by suspension culture.

For the adherent culture, a culture container, for example, a dish, a flask, a microplate, or a cell culture sheet such as OptiCell (product name) (Nunc), is used.

The container for use in adherent culture may be surface-treated in order to improve adhesiveness to cells (hydrophilicity), or coated with a substrate for cell adhesion such as collagen, gelatin, poly-L-lysine, poly-D-lysine, laminin, fibronectin, Matrigel, or vitronectin. A container without such surface treatment or coating is more preferably used.

In the suspension culture, the cells are dispersed into a medium, and an aggregated cell mass is formed while medium components and the internal oxygen concentration of the medium are uniformized by stirring or shaking. The suitable stirring rate is appropriately set according to a cell density and the size of a culture container. Excessive stirring or shaking places physical stress on the cells and inhibits aggregated cell mass formation. Thus, the stirring or shaking rate is controlled so as to be able to uniformize medium components and the internal oxygen concentration of the medium and so as not to inhibit aggregated cell mass formation. The suspension culture may be performed by still standing without stirring or shaking.

For the suspension culture, it is preferred to use a container with low-adhesion coating such as Prime surface (product name, Sumitomo Bakelite Co., Ltd.).

The culture temperature is not particularly limited and is 30 to 40°C (for example, 37°C). A carbon dioxide concentration in the culture container is on the order of, for example, 5%.

The production of an intermediate mesoderm and a kidney organoid is confirmed by, for example, a method of measuring the expression of a marker protein or a marker gene.

Provided that the obtained cell mass expresses OSR1, the cell mass can be determined as an intermediate mesoderm.

Provided that the obtained cell mass has the ability to produce EPO under hypoxic conditions and expresses renal stromal, glomerular and renal tubular markers, the cell mass can be determined as a kidney organoid. The renal stromal cell marker includes FOXD1, PDGFRβ and CD73. The glomerular marker includes WT1 and NPHS1. The renal tubular marker includes LTL, CUBN and E-cadherin.

Also, the production of an intermediate mesoderm may be confirmed, for example, by confirming the ability to induce the differentiation of the cells into glomerulus or renal tubule.

### 3. Application

The renal stromal cells and the renal stromal precursors and the kidney organoid obtainable by the production methods according to the present invention may be used in the pathological analysis of renal disease and may be applied to the screening for drug discovery for renal disease and the evaluation test of renal toxicity, etc.

### 3-1. Method for screening for substance for prevention or treatment of renal fibrosis

The present invention also provides a method for screening for a substance for the prevention or treatment of renal fibrosis, comprising the steps of:
(1) culturing renal stromal cells obtained by the production method mentioned above in the presence of a substance that induces the fibrosis of the cells to provide a cell population comprising fibrotic renal stromal cells;
(2A) culturing the cell population in the presence of and in the absence of a test substance;
(3A) measuring a degree of fibrosis of the cells in each of the cell populations thus obtained; and
(4A) selecting a test substance that has reduced the degree of fibrosis of the cells in the cell population cultured in the presence of the test substance as compared with the cell population cultured in the absence of the test substance.

In the step (1), renal stromal cells obtained by the step (3) in the method for producing renal stromal cells mentioned above can be further cultured in a medium supplemented with a fibrosis-inducing substance to obtain a cell population comprising fibrotic renal stromal cells.

The cell density of the cell population of the renal stromal cells for use in screening is not particularly limited and, in the case of adherent culture, can be, for example, 5,000 to 500,000 cells/cm², preferably 10,000 to 200,000 cells/cm², more preferably 20,000 to 100,000 cells/cm², particularly preferably 50,000 cells/cm². In the case of suspension culture, the cell density can be, for example, 5 to 500 cells/µL, preferably 20 to 200 cells/µL, particularly preferably 50 to 100 cells/µL.

The substance that induces the fibrosis of the renal stromal cells (fibrosis-inducing substance) can be, for example, a member of the TGFβ family and is preferably TGFβ. A particularly preferred isoform is TGFβ1.

The concentration of the fibrosis-inducing substance and the culture period may be appropriately set according to the substance used. In the case of using, for example, TGFβ1, the concentration is 0.1 to 100 ng/ml, preferably 1 to 30 ng/ml, particularly preferably about 10 ng/ml, and the culture period is 2 to 20 days, preferably 4 to 14 days, particularly preferably about 9 days.

In the present invention, it has been revealed that when TGFβ1 is removed from a medium after induction of fibrosis at a concentration of 1 ng/ml or lower, fibrotic renal stromal cells are restored to a non-fibrotic state. The fibrosis of the renal stromal cells can be maintained even after removal of TGFβ1 from the medium by inducing the fibrosis with TGFβ1 at a concentration of 10 ng/ml or higher. Therefore, a substance that improves a sustained fibrotic state can be screened for.

The test substance for use in the step (2A) and its concentration may be appropriately selected. In this step, a substance known to reduce the degree of fibrosis of renal stromal cells can be used as a positive control. For example, a TGF receptor inhibitor (SB431542, etc.) is used as the positive control.

The measurement of the degree of fibrosis of the cells in the step (3A) can be performed, for example, by determining the proportion of an area or a volume occupied by fibrotic cells in a given two-dimensional or three-dimensional region.

When the degree of fibrosis of the cells is reduced in the cell population cultured in the presence of the test substance as compared with the cell population cultured in the absence of the test substance, it is possible that the test substance is useful for improving renal fibrosis.

The proportion of fibrotic cells can be determined, for example, by fluorescently labeling the whole cells through the expression of a reporter protein (for example, tdTomato) under the control of a promoter of house keeping gene (for example, GAPDH), and detecting the fluorescence to measure the area or volume of the cells. It is known that the proliferation of fibroblasts present in renal stroma is promoted by fibrosis and both the area and the volume of each individual cell are also increased. The degree of fibrosis of the renal stromal cells can also be measured on the basis of the increased area or volume of the cells.

The area and volume of the fluorescently labeled cells can be measured by a known method. For example, an image of the cells fluorescently photographed is converted to 8 bits for binarization so that the cell area can be measured. The fluorescently taken image can be analyzed using, for example, image analysis software Image J (NIH). The degree of fibrosis can be evaluated by determining the proportion of the area of reporter protein-positive cells to the given area to be measured.

The degree of fibrosis can be measured, for example, by inoculating a given number of renal stromal cells into each well, adding a test substance thereto, culturing the cells, and then comparing a cell area within the well with a cell area within a well without the addition of the test substance. For example, provided that the cell area of the well supplemented with the test substance is smaller than that of the well without the addition of the test substance, the test substance can be evaluated as having reduced the degree of fibrosis. Alternatively, provided that that the cell area of the well supplemented with the test substance is larger than that of the well without the addition of the test substance, the test substance can be evaluated as having elevated the degree of fibrosis. The rates of reduction and increase in cell area are calculated according to the expression "(Cell area in the presence of the test substance) / (Cell area in the absence of the test substance) × 100 (%)".

The degree of fibrosis of the cells can also be measured by determining the proportion of αSMA-positive cells to the cell population through immunostaining using an antibody against αSMA. The proportion of αSMA-positive cells can be measured by a known method.

3-2. Method for determining biomarker for renal fibrosis

The present invention also provides a method for determining a biomarker for renal fibrosis, comprising the steps of:
(1) culturing renal stromal cells obtained by the production method mentioned above in the presence of a substance that induces the fibrosis of the cells to provide a cell population comprising fibrotic renal stromal cells;
(2B) identifying a substance contained in the culture solution of the cell population; and
(3B) comparing the identified substance with a substance contained in a body fluid of a mammal having renal fibrosis to determine an identical substance.

The step (1) is the same as that of the screening method in the preceding section.

The identification of the substance contained in the culture solution in the step (2B) can be performed, for example, by recovering a protein component in a supernatant by acetone precipitation or the like, and determining the amino acid sequence of a peptide fragment obtained by treatment with protease such as trypsin. The amino acid sequence of the peptide fragment can be determined by liquid chromatography mass spectrometry.
It is preferred for the protein component in the supernatant to remove a major protein component (albumin, IgG, IgA, etc.) before acetone precipitation.

Information on the substance contained in the body fluid of a mammal having renal fibrosis (diseased individual) for the step (3B) can be obtained from a database such as the Japan Chronic Kidney Disease Database (J-CKD-DB) provided by the Japanese Society of Nephrology (JSN) and the Japan Association for Medical Informatics (JAMI). For example, urine, blood, serum, plasma, saliva, perspiration, lymph, tissue fluid, extracellular fluid, or intracellular fluid can be used as the body fluid. The information may be obtained by the proteome analysis of urine, biopsy (examination of collected living tissues), blood, serum or plasma, etc. from the diseased individual.

It is possible that the substance that is secreted into the culture solution of the fibrotic renal stromal cells and is also contained in the body fluid of the diseased individual can be used as a biomarker for renal fibrosis.

### 3-3. Medicament

The renal stromal cells and the renal stromal precursors and the kidney organoid obtained by the production methods according to the present invention may serve as a cell preparation for the prevention or treatment of renal disease.

Specifically, the present invention also provides a medicament comprising renal stromal precursors derived from pluripotent stem cells and/or renal stromal cells derived from pluripotent stem cells and/or a kidney organoid, and a method for producing the same.

The method for producing the medicament according to the present invention may comprise the steps (1) to (3) of the method for producing renal stromal cells according to the present invention.

Examples of the renal disease include acute glomerulonephritis, chronic glomerulonephritis, rapidly progressive glomerulonephritis, diabetic nephropathy, and nephrogenic anemia.

The renal stromal cells, the renal stromal precursors or the kidney organoid contained in the medicament may be, for example, cells recovered by detaching cells during culture, or may be cells frozen in a cryopreservation solution.

The medicament may contain other components such as a pharmaceutically acceptable carrier or additive appropriate for a purpose or a form according to a routine method. Examples of the carrier or the additive include tonicity agents, thickeners, sugars, sugar alcohols, antiseptics (preservatives), germicides or antimicrobial agents, pH adjusters, stabilizers, chelating agents, oil bases, gel bases, surfactants, suspending agents, fluidizers, dispersants, buffers, and antioxidants.

The medicament provides a method for preventing or treating renal disease, comprising administering a therapeutically effective amount of the medicament to a subject.

The therapeutically effective amount is an amount that can produce a therapeutic effect on the disease by the administration of the medicament to a patient as compared with a control without the administration. Specifically, the therapeutically effective amount may be appropriately set depending on the dosage form of the medicament, an administration method, the purpose of use, and the age, body weight, symptoms, etc. of a patient. The effective amount per course of treatment in a human (for example, an adult human) is, for example, 200,000 to 1,00,000,000 cells/kg body weight in terms of the number of cells. These cells may be dispersed in a state of single cells, may be a cell mass (sphere) in which a plurality of cells have gathered, or may be a mixture thereof.

Examples of the method for administering the medicament include intraperitoneal injection, direct injection to the kidney by opening the abdominal cavity, and the attachment of a sheet-like device containing the cells around the kidney.

The present invention also provides a frozen stock comprising renal stromal cells, renal stromal precursors or a kidney organoid obtained by the production method mentioned above.

The frozen stock can be produced by separating the obtained renal stromal cells, renal stromal precursors or kidney organoid from the medium by centrifugation, and suspending the cells or the kidney organoid in a cryopreservation solution for freezing. A conventional reagent for use in the cryopreservation of cells can be used as the cryopreservation solution. For example, Cryostem Freezing Medium (trade name) and Stemcell Banker GMP Grade (Nippon Zenyaku Kogyo Co., Ltd.) are commercially available.

The frozen stock of the renal stromal precursors may be used as a starting material for causing the differentiation of renal stromal precursors to obtain renal stromal cells and a kidney organoid. Also, the frozen stock of the renal stromal cells or the kidney organoid may be used for preparing tissue models having renal stromal cells or a kidney organoid as a constituent.

### Examples

### [Example 1: Induction of hNCCs from iPSCs]

### Example 1-1: Induction of neural crest cells (NCCs) from induced pluripotent stem cells (iPSCs)

NCCs were induced from human iPSCs under xeno-free conditions according to the method described in Non

### Patent Literature 4.

The human iPSCs used were a 1231A3 line (see Non Patent Literature 5).

The human iPSCs were inoculated at 2 × 10⁵ cells/dish to a 10 cm dish coated with Laminin-511 (iMatrix-511 Silk, Nippi, Inc., #387-10131) (0.5 g/cm²), and precultured for 24 hours in StemFit AK03N (A+B+C) medium (Ajinomoto Co., Inc.) supplemented with 10 µM Y-27632 (FUJIFILM Wako Pure Chemical Corp., #034-24024).

The medium was replaced with a medium containing no Y-27632, and the human iPSCs were cultured for 4 days to form colonies (Days -4 to 0).

The human iPSCs that formed colonies were cultured for 10 days (Days 0 to 10) in StemFit AK03N (A+B) medium supplemented with 10 µM SB431542 (FUJIFILM Wako Pure Chemical Corp., #035-24294) and 1 µM CHIR99021 (Axon Medchem, #1386) to obtain SOX10-positive NCCs.

### Example 1-2: Posterior shift of NCCs with retinoic acid (RA) (induction of hNCCs)

From Day 6 during the culture of Example 1-1, 1 µM RA was added to the medium for the purpose of posteriorly shifting NCCs, and the cells were cultured up to Day 14 (Days 0 to 14). On Day 14, the expression of erythropoietin (EPO) was confirmed at the mRNA level and the protein level.

The expression of EPO was confirmed at both the mRNA level and the protein level. An immunohistochemical staining image of EPO and SOX10 is shown in Figure 1.

### Example 1-3: Study on induction period of differentiation

The induction and posterior shift of neural crest cells were performed in the same manner as in Examples 1-1 and 1-2 except that the concentration of RA to be added to the medium was changed to 0, 1 or 3 µM. The amount of EPO secreted into the medium in an induction period was quantified (EPO ELISA Kit, Sigma-Aldrich Co. LLC, #1693417) under the condition of each RA concentration (0, 1 or 3 µM).

Change in the accumulation of EPO into the medium after the start of induction of differentiation is shown in Figure 2. The secretion of EPO into the medium was confirmed from Day 9, and the accumulation was maximized on Day 18. This tendency was found irrespective of the RA concentration, whereas the amount of EPO secreted was largest under the condition with the RA concentration of 3 µM.

### Example 1-4: Confirmation of hypoxic response of posteriorly shifted NCCs (hNCCs)

EPO-producing cells are known to perform the production and secretion of EPO in response to HIF signals in a hypoxic environment. The hypoxic response of the hNCCs on Day 18 prepared in Example 1-3 was confirmed using two types of compounds that activated HIF signals (FG-2216: Selleck Chemicals #18382 and FG-4592: Selleck Chemicals #S1007, 100 µM each). Results of quantifying the amount of EPO secreted into the medium on 1 day after addition of FG-2216 or FG-4592 are shown in Figure 3. The secretion of EPO into the medium was increased in response to hypoxic stimulation.

### Example 1-5: Study on RA concentration

The induction of NCCs from iPSCs and posterior shift thereof were performed in the same manner as in Examples 1-1 and 1-2 except that the concentration conditions of RA to be added to the medium were changed to the range of 0 to 10 µM; and the culture period was prolonged from Day 10 to Day 21. The expression of the hindbrain marker HOXB4 was confirmed at the mRNA level. Also, the amount of EPO secreted into the medium was measured.

Results of measuring the expression level of HOXB4 on Day 10 are shown in Figure 4. The expression level of HOXB4 was increased in a RA concentration-dependent manner. Results of measuring the amount of EPO secreted on Day 18 are shown in Figure 5. The amount of EPO secreted was largest under the RA concentration condition of 3 µM.

### [Example 2: Induction of RSPs from hNCCs]

### Example 2-1: Establishment of EPO-emGFP knock-in human iPS cell line

An EPO-emGFP knock-in human iPS cell line was prepared which bicistronically expressed EPO and GFP (emGFP) under the control of EPO promoter.

A 1231A3 line was cotransfected with a donor vector with the stop codon site of the EPO gene substituted with F2A-emGFP, sgRNA expression plasmid of the target sequence and SpCas9 D10A nickase expression plasmid (FUJIFILM Wako Pure Chemical Corp.) using Neon Transfection system (Life Technologies Corp.).

An EPO-emGFP line in which the gene of interest was introduced in the chromosome was selected by drug selection using Geneticin (Invitrogen Corp.: #10131035) and PCR.

Further, the sequence of the drug selection moiety of the EPO-emGFP line was removed using Excision only piggyBAC transposase (Transposagen Biopharmaceuticals, Inc.: #PBx-m) to obtain an EPO-emGFP knock-in human iPS cell line.

### Example 2-2: Sorting of EPO-producing hNCCs using EPO-emGFP knock-in human iPS cell line

hNCCs were induced by the method described in Example 1 using the EPO-emGFP knock-in human iPS cell line established in Example 2-1. EPO-expressing cells were separated (sorted) from the hNCCs on Day 18 using a cell sorter (BD, FACSAria II). Specifically, hNCCs prepared as single cells were suspended in a HBSS solution (FUJIFILM Wako Pure Chemical Corp.) containing 2% BSA (Sigma-Aldrich Co. LLC, A8806-5G), and DAPI (Invitrogen Corp.: D1306)-negative (which indicates live cells) and emGFP fluorescence-positive cells were separated. Figure 6 shows results of measuring an EPO mRNA expression level in the EPO-emGFP knock-in human iPS cell line (before induction), hNCCs before sorting (in the drawing, "not sort"), GFP-negative hNCCs ("GFP-sort") and GFP-positive hNCCs ("GFP+ sort"). In the drawing, the ordinate shows the EPO mRNA expression level by a relative value with the expression level in the EPO-emGFP knock-in human induced pluripotent stem cell line (before induction) defined as 1. The high expression of EPO mRNA was confirmed in the GFP-positive hNCCs.

### Example 2-3: Induction of renal stromal precursors (RSPs) from hNCCs

The hNCCs (GFP-positive cells) obtained in Example 2-2 were inoculated at 10,000 cells/well to Prime Surface plate 96V (Sumitomo Bakelite Co., Ltd., MS-9096V) and suspension-cultured for 3 days (Days 18 to 21) in StemFit AK03N (A+B) medium supplemented with 10 µM SB431542, 3 µM CHIR99021, 40 ng/ml bFGF (FUJIFILM Wako Pure Chemical Corp., #068-04544), and 40 ng/ml EGF (FUJIFILM Wako Pure Chemical Corp., #053-07871).

The obtained cell mass was inoculated at 1 cell mass/well to a 6-well plate coated with fibronectin (MERCK, FC010-10MG), and cultured for 34 days (Days 21 to 55) in StemPro MSC SFM XenoFree medium (Thermo Fisher Scientific, Inc., #A1067501) to obtain CD73/PDGFRβ/FOXD1-positive RSPs.

### [Example 3: Induction of renal stromal cells (RSCs) from RSPs]

### Example 3-1: Induction of differentiation into RSCs

The RSPs obtained in Example 2 were inoculated at 2 × 10⁵ cells/well to a 6-well plate coated with fibronectin, and cultured for 15 days in STEMdiff APEL2 medium (STEMCELL Technologies Inc., ST-05275) to obtain RSCs (Days 55 to 70).

The hypoxia-responsive ability to produce EPO was confirmed as to the obtained RSCs.

EPO-producing cells are known to perform the production and secretion of EPO in response to HIF signals in a hypoxic environment. The RSCs were treated with a compound that activated HIF signals (FG-4592, Selleck Chemicals, #S1007, 100 µM).

Figure 7 shows results of measuring an EPO mRNA expression level in the cells on Day 18 (hNCCs; in the drawing, "NCC D18")), the cells on Day 39 (RSPs; "RSP D39"), the cells on day 15 of culture in STEMdiff APEL2 medium (Day 70) (RSCs; "APEL2 D15") and the hypoxically stimulated RSCs ("+FG4592"). In the drawing, the ordinate shows the EPO mRNA expression level by a relative value with the expression level in the EPO-emGFP knock-in human iPS cell line (before induction) defined as 1. Figure 8 shows an immunohistochemical staining image of EPO in RSCs under hypoxic conditions. The expression of EPO in response to hypoxic stimulation was found at the mRNA level and the protein level.

### Example 3-2: Influence of bFGF, FGF9 and PDGF-BB on differentiation into RSCs

The number of cells and the amount of EPO secreted into the medium which were obtained by adding 40 ng/ml bFGF, 200 ng/ml FGF9 (FUJIFILM Wako Pure Chemical Corp., #AF-100-23), and 10 ng/ml PDGF-BB (PeproTech, Inc., #100-14B) each alone or in combination to a medium were measured under the induction conditions of Example 3-1. EPO in the medium was measured by ELISA.

The results are shown in Figure 9. The addition of PDGF-BB increased the amount of EPO secreted, and the addition of bFGF promoted the proliferation of the cells. Furthermore, the proliferation of the cells and the increased amount of EPO secreted were found in the medium containing all of these factors.

### Example 3-3: Evaluation of renal stromal cell marker expression

The RSCs obtained in Example 3-2 were hypoxically stimulated (100 µM, FG-4592), and the expression of renal stromal cell markers (desmin, vimentin, CD73 and PDGFRβ) was confirmed at the mRNA level. The expression of all the markers was found (see Figure 10). In the drawing, "NHMC" depicts an expression level in commercially available normal human mesangial cells, and "Kidney" depicts an expression level in a commercially available renal cDNA library.

### Example 3-4: Evaluation of TGFβ1 response

*In vivo* renal stromal cells are known to cause differentiation into αSMA-positive myofibroblasts and hypertrophy (so-called fibrosis) in response to TGFβ1 stimulation. RSCs were obtained by the methods described in Examples 1, 2 and 3-1 from the EPO-emGFP/GAPDH-tdTomato (GAPDH-tdT) dual knock-in human iPS cell line obtained in Example 4-2 mentioned later. In the method described in Example 1, the RA concentration was set to 3 µM. RSCs on day 4 of culture (D4) were treated with TGFβ1 (0.01 to 10 ng/ml) for 9 days (D13), and the degree of fibrosis was evaluated by determining the ratio of tdTomato-positive cells using image analysis software Image J (NIH). Specifically, a fluorescently taken image was converted to 8 bits for binarization, and the whole area was divided into "area of cells" and "other areas (areas containing no cell)". The proportion was calculated according to the expression "Area of cells" / "Whole area".

The results are shown in Figures 11 and 12. Differentiation into αSMA-positive myofibroblasts and cellular hypertrophy by TGFβ1 stimulation were found . The cellular hypertrophy was caused in a TGFβ1 concentration-dependent manner and suppressed by cotreatment with a TGF receptor inhibitor (SB431542) (see Figure 12).

### Example 3-5: Screening for prophylactic or therapeutic drug for renal fibrosis

Example 3-4 indicated that RSCs become fibrotic in response to TGFβ1. RSCs, the fibrosis of which was induced by 1 ng/ml TGFβ1, were restored to non-fibrotic RSCs by removing TGFβ1 from the medium (Figure 13). By contrast, RSCs, the fibrosis of which was induced by 10 ng/ml TGFβ1, maintained the fibrotic state even after removal of TGFβ1 from the medium (Figure 14). By exploiting this property of fibrosis of RSCs, a screening system for a compound that improved a sustained fibrotic state was constructed.

RSPs were inoculated at 3000 cells/well to a 384-well plate, and the induction of RSCs was started in STEMdiff APEL2 medium (STEMCELL Technologies Inc., ST-05275). On day 4 of culture (D4), fibrosis was induced by the addition of 10 ng/ml TGFβ1. During the culture period, the culture solution containing TGFβ1 was replaced with a fresh one every 3 days. On day 13 of culture (D13), TGFβ1 was removed from the medium, and culture was further continued for 2 days (D15). The TGF receptor inhibitor SB431542 (SB) was added as a test substance to the medium of the cells on D15, and the cells were further cultured for 9 days (D24). Then, the area of tdTomato-positive cells was determined.

The results are shown in Figure 15. Enhanced fibrosis was seen on D15 or later in a control without the addition of SB (Cont). On the other hand, the marked suppression of fibrosis was able to be confirmed in the group supplemented with SB (SB+). It was able to be confirmed that this screening system is useful in screening for a compound having the activity of improving renal fibrosis.

### Example 3-6: Method for searching for biomarker related to renal fibrosis

A biomarker related to renal fibrosis was searched for by analyzing components in a culture supernatant of RSCs with induced fibrosis.

RSPs were inoculated at 2 × 10⁶ cells/dish to a 10 cm dish, and the induction of RSCs was started in STEMdiff APEL2 medium (STEMCELL Technologies Inc., ST-05275). On day 7 of culture (D7), 10 ng/ml TGFβ1 was added thereto, and the cells were further cultured for 9 days to induce fibrosis.

The culture supernatant was recovered. After removal of albumin, IgG and IgA, a peptide fragment was obtained by acetone precipitation and trypsin digestion. The amino acid sequence of the peptide fragment was determined by LC/MS analysis.

As a result, a peptide fragment of fibronectin (FN1) widely known as a fibrosis marker was detected, and FN1 was detected by about 10 times in the culture supernatant of the cells treated with TGFβ1 as compared with the culture supernatant of untreated cells.

### [Example 4: Preparation of kidney organoid (Mini-kidney)]

### Example 4-1: Induction of differentiation of iPSCs into intermediate mesoderm (IM)

The induction of differentiation of iPSCs into IM and the induction of differentiation of IM and the RSPs obtained in Example 2 into a kidney organoid were performed with reference to Non Patent Literatures 6 and 7.

### The human iPSCs used were a 1231A3 line.

The human iPSCs were inoculated at 5.76 × 10⁵ cells/well to a 6-well plate coated with Laminin-511, and precultured for 24 hours in StemFit AK03N (A+B+C) medium supplemented with 10 µM Y-27632.

The cells were cultured for 4 days in a medium obtained by adding 8 µM CHIR99021 to APEL2 medium supplemented with 5% PFHM-II (Thermo Fisher Scientific, Inc., #1204007) (hereinafter, also referred to as "IM induction basal medium"). Next, the cells were cultured for 3 days in IM induction basal medium supplemented with 200 ng/ml FGF9 and 1 µg/ml heparin (FUJIFILM Wako Pure Chemical Corp., #081-00136) to obtain IM (Day 7).

### Example 4-2: Establishment of EPO-emGFP/GAPDH-tdTomato (GAPDH-tdT) dual knock-in human iPS cell line

A fusion protein of GAPDH and tdTomato (GAPDH-tdT) to be expressed under the control of GAPDH promoter was further knocked in the EPO-emGFP knock-in human iPS cell line established in Example 2-1.

The EPO-emGFP knock-in human iPS cell line was cotransfected with a donor vector with the stop codon site of the GAPDH gene substituted with F2A-tdT, and a complex of sgRNA (Integrated DNA Technologies, Inc.) of the target sequence and SpCas9 D10A nickase protein (Integrated DNA Technologies, Inc., #1081062) using Neon Transfection system (Life Technologies Corp.).

Cells were selected on the basis of the fluorescence of tdT, and cells having an insert of the gene of interest in the chromosome was obtained as an EPO-emGFP/GAPDH-tdT dual knock-in human iPS cell line by PCR.

### Example 4-3: Coculture of RSPs and IM

The IM on Day 7 of Example 4-1 was detached with Accutase (Innovative Cell Technologies, Inc., AT104). Also, RSPs were induced by the methods described in Examples 1 and 2 from the EPO-emGFP/GAPDH-tdT dual knock-in human iPS cell line prepared in Example 4-2.

The IM and the RSPs (tdT-labeled) were mixed to prepare a cell mass (pellet), which was then inoculated onto Transwell (Corning Inc., #3450) and cocultured. 5 × 10⁵ cells/pellet of the IM were mixed with a 1/5 amount (1 × 10⁵ cells/pellet) or a 1/25 amount (2 × 10⁴ cells/pellet) of the RSPs.

The cell mass (pellet) was treated for 1 hour with IM induction basal medium supplemented with 5 µM CHIR99021 and 1 µg/ml heparin, and then cultured for 5 days in IM induction basal medium supplemented with 200 ng/ml FGF9 and 1 µg/ml heparin. Then, the medium was replaced with IM induction basal medium supplemented with 1 µg/ml heparin, followed by further culture for about 3 weeks to obtain a kidney organoid (Mini-kidney). A microscope image of the kidney organoid is shown in Figure 16.

### Example 4-4: Evaluation of ability of Mini-kidney to produce EPO under hypoxic conditions

RNA was extracted from the kidney organoid on Day 25 from the start of coculture (Mini-kidney, IM+RSP (D25)) obtained in Example 4-3 and a kidney organoid containing no RSP (KiO (D25)), and libraries for transcriptome analysis were prepared using Ion AmpliSeq(TM) Transcriptome Human Gene Expression Kit (Thermo Fisher Scientific, Inc.: #A26326). The prepared libraries were subjected to sequencing analysis (Ion S5 XL sequencer, Thermo Fisher Scientific, Inc.) using Ion 54(TM) Chip Kit (Thermo Fisher Scientific, Inc., #A27765) to quantify the expression level of EPO mRNA.

The results are shown in Figure 17. The significant expression of EPO mRNA was not seen in any of an iPS cell line, IM, and the kidney organoid containing no RSP (KiO (D25)). By contrast, the significant expression of EPO mRNA was confirmed in the Mini-kidney (IM+RSP (D25)). As a result of confirming the hypoxic response of the Mini-kidney in the same manner as in Example 1-4, the EPO expression level was increased by hypoxic stimulation (100 µM FG-4592) (IM+RSP (D25) + FG4592).

### Example 4-5: Evaluation of renal stromal cell marker in Mini-kidney

The mRNA expression levels of renal stromal cell markers (desmin, vimentin, PDGFRβ and CD73) were quantified by the sequencing analysis of the libraries for transcriptomes prepared in Example 4-4.

The results are shown in Figure 18. The expression of all the renal stromal cell markers was confirmed in the Mini-kidney (IM+RSP (D25)). As a result of confirming the hypoxic response of the Mini-kidney (IM+RSP (D25)) in the same manner as in Example 1-4, the expression levels of desmin and CD73 were decreased by hypoxic stimulation (100 µM FG-4592) (IM+RSP (D25) + FG4592) .

### Example 4-6: Evaluation of glomerular marker and renal tubular marker in kidney organoid (Mini-kidney)

The expression of the glomerular cell marker WT1 and the renal tubular cell marker LTL was detected by immunostaining. It was confirmed that WT1-positive cells and LTL-positive cells are adjacently localized (see Figure 19).

### Example 4-7: Evaluation of cell population of Mini-kidney

The kidney organoid on Day 25 from the start of coculture (Mini-kidney, IM+RSP (D25)) obtained in Example 4-3 and a kidney organoid containing no RSP (KiO (D25)) were dissociated into single cells. The preparation of cDNA libraries, the determination of nucleotide sequences and the creation of a T-SNE plot were performed using a single-cell analysis platform (Chromium Controller: 10X Genomics: #1000202).

The T-SNE plot is shown in Figure 20. The population of RSPs as well as the population of RSCs was increased in the kidney organoid containing RSPs (IM+RSP (D25); right diagram) as compared with the kidney organoid containing no RSP (KiO (D25); left diagram), indicating that the differentiation of RSPs into RSCs is promoted by coculture with IM. In the population of RSCs, it was able to be confirmed that the expression of NELL2, PAX7, NGFR and NRK was elevated.

### [Example 5: In vivo transplantation experiment of RSPs]

In order to confirm the *in vivo* functions of RSPs, the RSPs were transplanted beneath the renal capsule of an immunodeficient NOG mouse (In-Vivo Science Inc.).

RSPs were induced according to the methods described in Examples 1 and 2 using the EPO-emGFP/GAPDH-tdTomato (GAPDH-tdT) dual knock-in human iPS cell line established in Example 4-2. The RSPs were inoculated at 30,000 cells/well to Prime Surface 96-well V bottom plate (Sumitomo Bakelite Co., Ltd. #MS-9096V) and cultured for 3 days to form a cell mass. The cell mass was transplanted beneath the left renal capsule of a NOG mouse. 35 days later, the cell mass-transplanted kidney was isolated, and the fluorescence of GAPDH-tdT was observed under a stereoscopic microscope (Leica Camera AG, MZ10F).

As a result, it was confirmed that cells migrated from the transplanted cell mass and engrafted in the kidney (see Figure 21). Furthermore, the fluorescence of EPO-emGFP was confirmed in some of the transplanted RSPs (GAPDH-tdT-positive cells) by fluorescent observation under a fluorescence microscope (Keyence Corp., BZ-700) (see Figure 22).

## Claims

1. A method for producing renal stromal cells, comprising a step (3) of culturing renal stromal precursors in a medium comprising a platelet derived growth factor receptor agonist to obtain renal stromal cells.

2. The production method according to claim 1, wherein the medium further comprises a basic fibroblast growth factor and/or fibroblast growth factor 9.

3. The production method according to claim 1 or 2, further comprising a step (2) of inducing renal stromal precursors from neural crest cells.

4. The production method according to any one of claims 1 to 3, further comprising a step (1) of culturing pluripotent stem cells in a medium comprising a GSK3β inhibitor, a TGFβ inhibitor, and retinoic acid and/or a derivative thereof to induce neural crest cells.

5. The production method according to claim 3 or 4, wherein the neural crest cells are hindbrain neural crest cells.

6. The production method according to any one of claims 1 to 5, wherein the renal stromal cells produce erythropoietin under hypoxic conditions.

7. The production method according to any one of claims 1 to 6, wherein the platelet derived growth factor receptor agonist is a platelet derived growth factor (PDGF) .

8. A medium for use in producing renal stromal cells, comprising a platelet derived growth factor receptor agonist.

9. The medium according to claim 8, further comprising a basic fibroblast growth factor and/or fibroblast growth factor 9.

10. The medium according to claim 8 or 9, wherein the platelet derived growth factor receptor agonist is a platelet derived growth factor (PDGF).

11. A method for producing a kidney organoid, comprising the step of coculturing renal stromal cells or renal stromal precursors, and an intermediate mesoderm.

12. The production method according to claim 11, wherein
the renal stromal cells or the renal stromal precursors are
obtained by a method for producing renal stromal cells, comprising a step (3) of culturing renal stromal precursors in a medium comprising a platelet derived growth factor receptor agonist to obtain renal stromal cells, or
obtained by a step (2) of inducing renal stromal precursors from neural crest cells.

13. A method for screening for a substance for the prevention or treatment of renal fibrosis, comprising the steps of:
culturing renal stromal cells obtained by a production method according to any one of claims 1 to 7 in the presence of a substance that induces the fibrosis of the cells to provide a cell population comprising fibrotic renal stromal cells;
culturing the cell population in the presence of and in the absence of a test substance;
measuring a degree of fibrosis of the cells in each of the cell populations thus obtained; and
selecting a test substance that has reduced the degree of fibrosis of the cells in the cell population cultured in the presence of the test substance as compared with the cell population cultured in the absence of the test substance.

14. A method for determining a biomarker for renal fibrosis, comprising the steps of:
culturing renal stromal cells obtained by a production method according to any one of claims 1 to 7 in the presence of a substance that induces the fibrosis of the cells to provide a cell population comprising fibrotic renal stromal cells;
identifying a substance contained in the culture solution of the cell population; and
comparing the identified substance with a substance contained in a body fluid of a mammal having renal fibrosis to determine an identical substance.

15. A method for producing a medicament for the prevention or treatment of kidney damage containing renal stromal precursors, the production method comprising a step (2) of inducing renal stromal precursors from neural crest cells.

16. A method for producing a medicament for the prevention or treatment of kidney damage containing renal stromal cells, the production method comprising a step (3) of culturing renal stromal precursors in a medium comprising a platelet derived growth factor receptor agonist to obtain renal stromal cells.

17. The production method according to claim 15 or 16, further comprising a step (1) of culturing pluripotent stem cells in a medium comprising a GSK3β inhibitor, a TGFβ inhibitor, and retinoic acid and/or a derivative thereof to induce neural crest cells.

18. A medicament comprising renal stromal precursors derived from pluripotent stem cells and/or renal stromal cells derived from pluripotent stem cells.

19. A prophylactic or therapeutic agent for kidney damage, comprising renal stromal precursors derived from pluripotent stem cells and/or renal stromal cells derived from pluripotent stem cells.

20. A method for preventing or treating kidney damage, comprising the step of administering renal stromal precursors derived from pluripotent stem cells and/or renal stromal cells derived from pluripotent stem cells to a subject.

21. Renal stromal precursors derived from pluripotent stem cells and/or renal stromal cells derived from pluripotent stem cells for use in the prevention or treatment of kidney damage.

22. Use of renal stromal precursors derived from pluripotent stem cells and/or renal stromal cells derived from pluripotent stem cells for the production of a medicament for the prevention or treatment of kidney damage.
